(19) Europäisches Patentamt
European Patent Office
Office européen des brevets

(11) Veröffentlichungsnummer: **0 322 810 A2**

(12) # EUROPÄISCHE PATENTANMELDUNG

(21) Anmeldenummer: 88121622.0

(51) Int. Cl.4: C07D 501/36 , A61K 31/545

(22) Anmeldetag: 23.12.88

Patentansprüche für folgende Vertragsstaaten: GR + ES.

(30) Priorität: 28.12.87 US 138410
07.10.88 US 255004

(43) Veröffentlichungstag der Anmeldung: 05.07.89 Patentblatt 89/27

(84) Benannte Vertragsstaaten: AT BE CH DE ES FR GB GR IT LI LU NL SE


(71) Anmelder: F. HOFFMANN-LA ROCHE & CO. Aktiengesellschaft
CH-4002 Basel(CH)

(72) Erfinder: Chan, Ka-Kong
33 Charles Street Hopatcong
N.J. 07843(US)
Erfinder: Keith, Dennis Dalton
8 Mendl Terrace Montclair
N.J. 07042(US)

(74) Vertreter: Lederer, Franz, Dr. et al
Patentanwält Dr. Franz Lederer Lucile
Grahnstrasse 22
D-8000 München 80(DE)


(54) **Cephalosporinderivate.**


(57) Die Erfindung betrifft Cephalosporinderivate der allgemeinen Formel

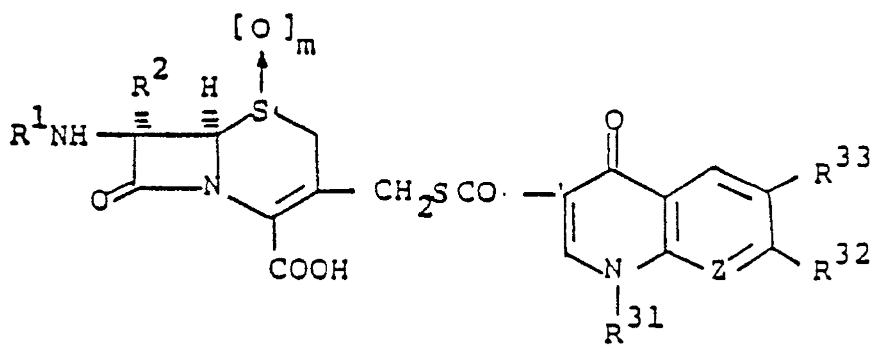

I

in der m 0, 1 oder 2, $R^1$ Wasserstoff oder eine Acylgruppe, $R^2$ Wasserstoff, niederes Alkoxy, niederes Alkylthio oder niederes Alkanoylamino, $R^{31}$ Wasserstoff, niederes Alkyl, niederes Alkenyl, $C_3$-$C_7$-Cycloalkyl, Halogen-niederes Alkyl oder Mono-, Di- oder Tri-halogenphenyl; Z $R^{30}$ -C oder Stickstoff, $R^{30}$ Wasserstoff oder Halogen, oder $R^{30}$ und $R^{31}$ zusammen eine $C_3$-$C_5$-Alkylengruppe, eine $C_2$-$C_4$-Alkylenmonooxygruppe oder eine $C_1$-$C_2$ Alkylendioxygruppe, $R^{32}$ Wasserstoff, Halogen, niederes Alkyl oder einen gegebenenfalls substituierten 5- oder 6-gliedrigen heterocyclischen Ring mit einem, zwei oder drei Sauerstoff-, Stickstoff- und/oder Schwefelatomen und $R^{33}$ Wasserstoff oder Halogen oder $R^{32}$ und $R^{33}$ zusammen eine $C_1$-$C_4$-Alkylendioxygruppe darstellen,
sowie leicht hydrolysierbare Ester und Salze dieser Verbindungen und Hydrate der Verbindungen der Formel I bzw. von deren Estern und Salzen.
Die Erfindung betrifft ebenfalls ein Verfahren zur Herstellung dieser Verbindungen sowie entsprechende pharmazeutische Präparate.


EP 0 322 810 A2

Die Produkte sind antimikrobiell wirksam.

# Cephalosporinderivate

Die vorliegende Erfindung betrifft Cephalosporinderivate der allgemeinen Formel

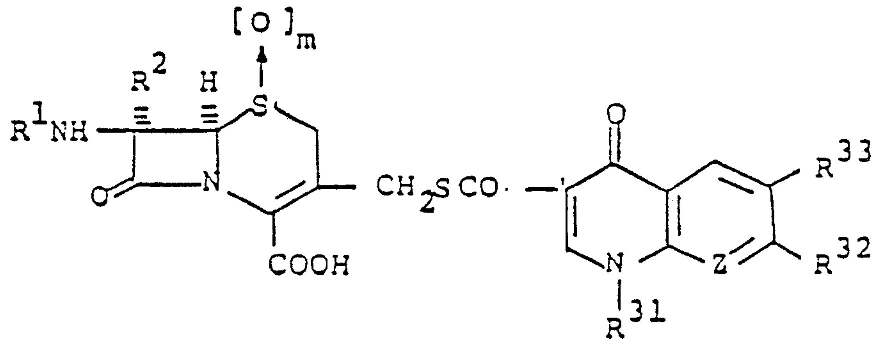

I

in der m 0, 1 oder 2, $R^1$ Wasserstoff oder eine Acylgruppe, $R^2$ Wasserstoff, niederes Alkoxy, niederes Alkylthio oder niederes Alkanoylamino, $R^{31}$ Wasserstoff, niederes Alkyl, niederes Alkenyl, $C_3$-$C_7$-Cycloalkyl, Halogen-niederes Alkyl oder Mono-, Di- oder Tri-halogenphenyl; Z $R^{30}$ -C oder Stickstoff, $R^{30}$ Wasserstoff oder Halogen, oder $R^{30}$ und $R^{31}$ zusammen eine $C_3$-$C_5$-Alkylengruppe, eine $C_2$-$C_4$-Alkylenmonooxygruppe oder eine $C_1$-$C_2$ Alkylendioxygruppe, $R^{32}$ Wasserstoff, Halogen, niederes Alkyl oder einen gegebenenfalls substituierten 5- oder 6-gliedrigen heterocyclischen Ring mit einem, zwei oder drei Sauerstoff-, Stickstoff- und/oder Schwefelatomen und $R^{33}$ Wasserstoff oder Halogen oder $R^{32}$ und $R^{33}$ zusammen eine $C_1$-$C_4$-Alkylendioxygruppe darstellen,
sowie leicht hydrolysierbare Ester und Salze dieser Verbindungen und Hydrate der Verbindungen der Formel I bzw. von deren Estern und Salzen.

In der obigen Formel I ist m vorzugsweise 0.

Der Ausdruck "niederes Alkyl" oder "Alkyl" bezieht sich auf gerad- und verzweigtkettige Kohlenwasserstoffgruppen mit 1-8, bevorzugt 1-4 Kohlenstoffatomen, z.B. Methyl, Aethyl, n-Propyl, Isopropyl, t-Butyl oder dergleichen.

Der Ausdruck "niederes Alkoxy" oder "Alkoxy" bezieht sich auf gerad- oder verzweigtkettige Kohlenwasserstoffoxygruppen, worin der Alkylrest die oben gegebene Bedeutung hat. Beispiele sind Methoxy, Aethoxy, n-Propoxy und dergleichen.

Der Ausdruck "Halogen" bezieht sich auf Chlor, Brom, Jod oder Fluor, sofern nicht anderweitig definiert.

Der Ausdruck "Aryl" bedeutet einen gegebenenfalls substituierten aromatischen Rest, wie z.B. Phenyl, Tolyl, Xylyl, Mesityl, Cumyl (Isopropylphenyl), Naphthyl und dergleichen, wobei die Arylgruppe z.B. 1-3 geeignete Substituenten tragen kann, wie Halogen (z.B. Fluor, Chlor, Brom), Hydroxy oder dergleichen.

Der Ausdruck "niederes Alkanoyl" oder "Alkanoyl" bezeichnet einen Rest der allgemeinen Formel
$R^{25}$-CO-
in der $R^{25}$ Wasserstoff oder $C_1$-$C_6$ Alkyl darstellt. Beispiele dieser Gruppen sind Acetyl, Formyl, Propionyl, n-Butyryl und dergleichen.

Der Ausdruck "substituiertes Phenyl" bedeutet eine mono- oder disubstituierte Phenylgruppe, wobei als Substituenten Halogen (z.B. Chlor, Brom, Fluor), niederes Alkyl, Amino, Nitro oder Trifluormethyl in Frage kommen.

Der Ausdruck "substituiertes Alkyl" bedeutet eine niedere Alkylgruppe, die z.B. durch Halogen (z.B. Chlor, Fluor, Brom), Trifluormethyl, Amino, Cyan etc. substituiert sein kann.

Der Ausdruck "niederes Alkenyl" oder "Alkenyl" bedeutet gerad- oder verzweigtkettige Kohlenwasserstoffgruppen mit einer olefinischen Doppelbindung und 2-6 Kohlenstoffatomen, d.h. den Rest von Verbindungen der Formel $C_nH_{2n}$, worin n 2-6 ist, z.B. Allyl, Vinyl etc.

Der Ausdruck "Aralkyl" bedeutet eine Kohlenwasserstoffgruppe mit aromatischer und aliphatischer Struktur, d.h. eine Kohlenwasserstoffgruppe, worin ein Wasserstoffatom der niederen Alkylgruppe durch eine monocyclische Arylgruppe substituiert ist, z.B. durch Phenyl, Tolyl etc.

Der Ausdruck "5-, 6 oder 7-gliedriger heterocyclischer Ring mit 1-4 Sauerstoff-, Stickstoff- und/oder Schwefelatomen" bedeutet z.B. die folgenden Gruppen: 6-gliedrige, Stickstoff-enthaltende heterocyclische Ringe, wie Pyridyl, Piperidyl, Piperidino, N-oxido-pyridyl, Pyrimidyl, Piperazinyl, Pyridazinyl, N-oxido-pyridazinyl, etc.; 5-gliedrige Stickstoff-enthaltende heterocyclische Ringe, z.B. Pyrrolidinyl, Pyrazolyl, Imidazolyl, Thiazolyl, 1,2,3-Thiadiazolyl, 1,2,4-Thiadiazolyl, 1,3,4-Thiadiazolyl, 1,2,3-Oxadiazolyl, 1,2,4-Oxadiazolyl, 1,3,4-Oxadiazolyl, 1,2,5-Oxadiazolyl, 1,2,3-Triazolyl, 1,2,4-Triazolyl, 1H-Tetrazolyl, 2H-Tetra zolyl etc. Diese heterocyclischen Ringe können weiter substituiert sein, wobei z.B. folgende Substituenten in Frage kommen: niederes Alkyl wie Methyl, Aethyl, n-Propyl, etc., niederes Alkoxy, z.B. Methoxy, Aethoxy, etc., Halogen wie Chlor, Brom, etc., Halogen-niederes Alkyl wie Trifluormethyl, Trichloräthyl, etc.. Amino, Mercapto, Hydroxy, Carbamoyl oder Carboxy, etc..

Der Ausdruck "Cyclo-niederes Alkyl" oder "Cycloalkyl" bedeutet einen 3-7-gliedrigen gesättigten carbocyclischen Rest, z.B. Cyclopropyl, Cyclobutyl, Cyclohexyl, etc..

$R^{30}$ und $R^{31}$ können zusammen $C_3$-$C_5$-Alkylen bedeuten, z.B. $-(CH_2)_3-$, $-(CH_2)_4-$, $-(CH_2)_5-$ oder $-CH(CH_3)-(CH_2)_2-$, ode auch $C_2$-$C_4$-Alkylenmonooxy, z.B. $-(CH_2)_2-O-$, $-(CH_2)_3-O-$, $-(CH_2)_4-O-$ oder $-CH(CH_3)-CH_2-O-$; oder auch $C_1$-$C_2$-Alkylendioxy, z.B. $-O-CH_2-O-$, $-O-(CH_2)_2-O-$ oder $-O-CH(CH_3)-O-$. Vorzugsweise wird ein 5- oder 6-gliedriger kondensierter Ring gebildet. $R^{32}$ und $R^{33}$ können zusammen $C_1$-$C_4$-Alkylendioxy bedeuten, z.B. $-O-CH_2-O-$, $-O-(CH_2)_2-O-$, $-O-(CH_2)_3-O-$, $-O-(CH_2)_4-O-$, $-O-CH(CH_3)-O-$, $-O-CH(CH_3)-CH(CH_3)-O-$ oder dergleichen. In diesem Falle wird vorzugsweise ein 5- oder 6-gliedriger kondensierter Ring gebildet.

Der Ausdruck "Acyl" (Substituent R') steht für alle organischen Reste, welche durch Entfernung der Hydroxygruppe aus einer Carbonsäure gebildet werden. Gewisse Acylreste sind bevorzugt: Beispiele solcher bevorzugten Acylgruppen sind diejenigen, welche bisher zur Acylierung von β-Lactamantibiotika, einschliesslich der 6-Aminopenicillansäure und deren Derivaten, sowie der 7-Aminocephalosporansäure und deren Derivaten, verwendet worden sind; siehe z.B. Cephalosporins and Penicillins, Verlag Flynn, Academic Press (1972), Belgische Patentschrift 866,038, veröffentlicht am 17. Oktober 1978, Belgische Patentschrift 867.994, veröffent licht am 11. Dezember 1978, U.S. Patentschrift 4,152,432, veröffentlicht am 1. Mai 1979, U.S. Patentschrift 3,971,778, veröffentlicht am 27. Juli 1976 und U.S. Patentschrift 4,173,199, veröffentlicht am 23. Oktober 1979. In diesen Publikationen werden verschiedene Acylreste, die im vorliegenden Falle Verwendung finden, erwähnt. Die nachfolgend aufgeführten Acylreste veranschaulichen weiter den Begriff Acyl, beschränkt ihn jedoch in keiner Weise. Beispiele für Acylreste sind:

a) aliphatische Gruppen der Formel

$R^5$-CO-

in der $R^5$ niederes Alkyl, $C_3$-$C_7$-Cycloalkyl, niederes Alkoxy, niederes Alkenyl, $C_3$-$C_7$-Cycloalkenyl oder Cyclohexadienyl; oder durch einen oder mehrere der Reste Halogen, Cyano, Nitro, Amino, Mercapto, niederes Alkylthio oder Cyanmethylthio substituiertes niederes Alkyl oder niederes Alkenyl darstellt;

(b) eine carbocyclische aromatische Gruppe der allgemeinen Formeln

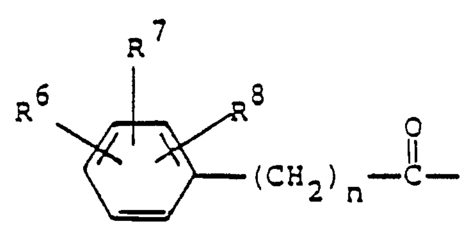

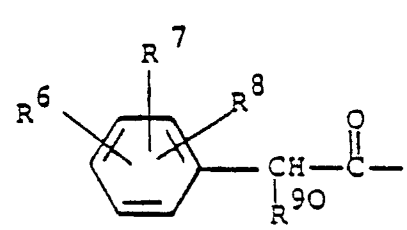

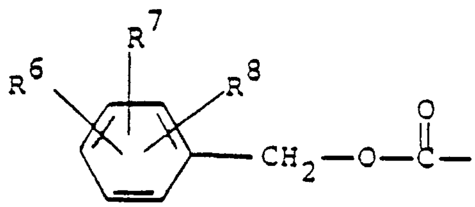

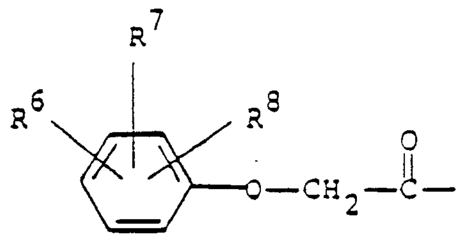

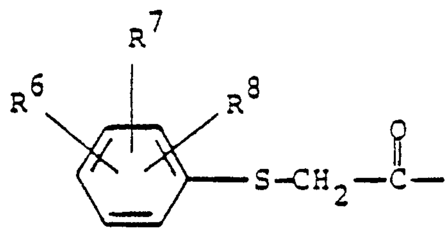

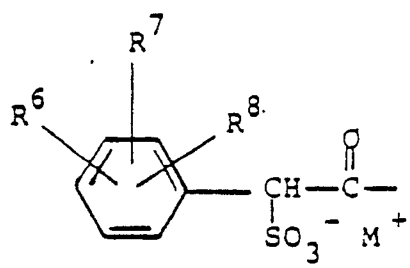

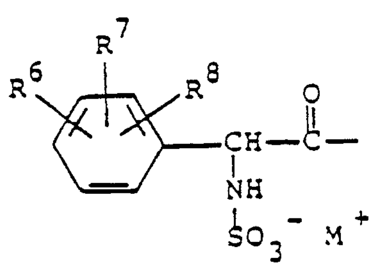

in der n 0, 1, 2 oder 3; $R^6$, $R^7$ und $R^8$ je Wasserstoff, Halogen, Hydroxy, Nitro, Amino, Cyano, Trifluormethyl, $C_1$-$C_4$-Alkyl, $C_1$-$C_4$-Alkoxy oder Aminomethyl, $R^{9c}$ Amino, Acylamino, Hydroxy, ein Carboxysalz, geschütztes Carboxy, wie z.B. Benzyloxycarbonyl, Formyloxy oder Azido und M ein Kation darstellen.

Bevorzugte carboxyclische aromatische Acylgruppen sind diejenigen der Formeln

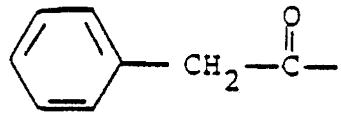

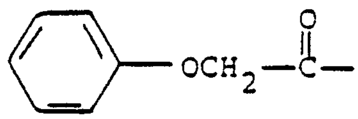

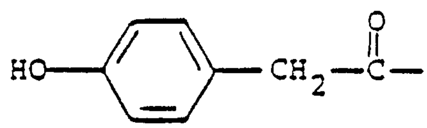

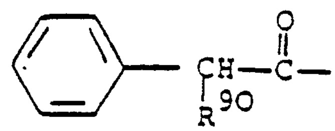

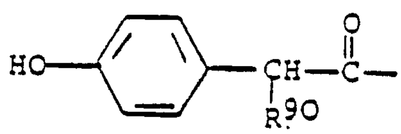

$R^{90}$ ist bevorzugt eine Aminogruppe, eine Hydroxygruppe oder ein Carboxy- oder Sulfosalz.

Beispiele anderer erfindungsgemäss einsetzbarer Acylgruppen sind:

Sulfophenylacetyl,
Hydroxysulfonyloxyphenylacetyl,
Sulfamoylphenylacetyl,
(Phenoxycarbonyl)phenylacetyl,
(p-Tolyloxycarbonyl)phenylacetyl,
Formyloxyphenylacetyl,
Carboxyphenylacetyl,
Formylaminophenylacetyl,
Benzyloxycarbonylphenylacetyl,
2-(N,N-Dimethylsulfamoyl)-2-phenylacetyl,
2-Brom-2-thienylacetyl, etc.

c) Heteroaromatische Gruppen der allgemeinen Formeln

$$R^{101}-(CH_2)_n-CO-$$

$$R^{101}-\underset{\substack{|\\ R^{90}}}{CH}-CO-$$

$$R^{101}-O-CH_2-CO-.$$

$$R^{101}-S-CH_2-CO-$$

$$R^{101}-CO-CO-$$

in der $R^{90}$ die oben gegebene Bedeutung hat, n 0, 1, 2 oder 3 und $R^{101}$ einen gegebenenfalls substituierten 5-, 6- oder 7-gliedrigen heterocyclischen Ring mit 1, 2, 3 oder 4 (vorzugsweise 1 oder 2) Stickstoff-, Sauerstoff- und/oder Schwefelatomen darstellt.

Beispiele heterocyclische Ringe sind Thienyl, Furyl, Pyrrolyl, Pyridinyl, Pyrazinyl, Thiazolyl, Pyrimidinyl und Tetrazolyl. Beispiele für Substituenten am heterocyclischen Ring sind Halogen, Hydroxy, Nitro, Amino, Cyano, Trifluormethyl, $C_1$-$C_4$-Alkyl sowie $C_1$-$C_4$-Alkoxy.

Bevorzugte heteroaromatische Acylgruppen sind diejenigen, worin $R^{101}$ 2-Amino-4-thiazolyl, 2-Amino-5-halogen-4-thiazolyl, 4-Aminopyridin-2-yl, 2-Amino-1,3,4-thiadiazol-5-yl, 2-Thienyl, 2-Furanyl, 4-Pyridinyl oder 2,6-Dichlor-4-pyridinyl bedeutet.

d) [[4-Substituierte-2,3-dioxo -1-piperazinyl)carbonyl]amino]arylacetylgruppen der Formel

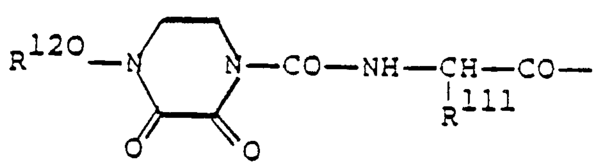

in der $R^{111}$ niederes Alkyl, Hydroxy-niederes Alkyl oder eine aromtische Gruppe der allgemeinen Formel

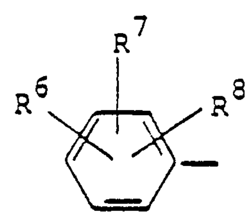

(in der $R^6$, $R^7$ und $R^8$ die obige Bedeutung haben) oder einen wie für $R^{101}$ definierten heterocyclischen Ring und $R^{120}$ gegebenenfalls durch einen oder mehreren der Reste Halogen, Cyan, Nitro, Amino und/oder Mercapto substituiertes niederes Alkyl darstellen, z.B. 4-niederes Alkyl (vorzugsweise Aethyl oder Methyl)-

2.3-dioxo-1-piperazincarbonyl-D-phenylglycyl.

e) (Substituierte Oxyimino)arylacetylgruppen der allgemeinen Formel

$$R^{130}-O-N=C-CO-$$
$$|$$
$$R^{101}$$

in der $R^{101}$ die obige Bedeutung hat und $R^{130}$ Wasserstoff, niederes Alkyl, $C_3$-$C_7$-Cycloalkyl, Carboxy-$C_3$-$C_7$-cycloalkyl oder substituiertes niederes Alkyl darstellt, wobei die niedere Alkylgruppe substituiert ist durch einen oder mehrere der Reste Halogen, Cyano, Nitro, Amino, Mercapto, niederes Alkylthio, eine durch $R^{111}$ oben definierte aromatische Gruppe, Carboxy (einschliesslich dessen Salze), niederes Alkanoylamino, niederes Alkoxycarbonyl, Phenylmethoxycarbonyl, Diphenylmethoxycarbonyl, Hydroxy(niederes Alkoxy)phosphinyl, Dihydroxyphosphinyl, Hydroxy(phenylmethoxy)phosphinyl oder di(niederes Alkoxy)-phosphinyl.

Beispiele für Reste

$$R^{130}-O-N=C-CO-$$
$$|$$
$$R^{101}$$

sind:

2-[(2-Chloracetamidothiazol-4-yl)]-2-[(p-nitrobenzyloxycarbonyl)methoxyimino]acetyl, 2-(2-Chloracetamidothiazol-4-yl)-2-methoxyiminoacetyl, 2-(2-Aminothiazol-4-yl)-2-isopropoxyiminoacetyl, 2-(2-Aminothiazol-4-yl)-2-methoxyiminoacetyl, 2-(2-Aminothiazol-4-yl)-2-hydroxyiminoacetyl, 2-Thienyl-2-methoxyiminoacetyl, 2-Furyl-2-methoxyiminoacetyl, 2-(4-Hydroxyphenyl)-2-methoxyiminoacetyl, 2-Phenyl-2-methoxyiminoacetyl, 2-Phenyl- 2-hydroxyiminoacetyl, 2-Thienyl-2-hydroxyiminoacetyl, 2-Thienyl-2-(dichloracetoxyimino)acetyl, 2-[4-(γ-D-Glutamyloxy)phenyl]-2-hydroxyiminoacetyl, 2-[4-(3-Amino-3-carboxypropoxy)phenyl]-2-hydroxyiminoacetyl, 2-(5-Chlor-2-chloracetamidothiazol-4-yl) -2-methoxyiminoacetyl, 2-(5-Chlor-2-aminothiazol-4-yl)-2-methoxyiminoacetyl, 2-[2-(t-Butoxycarbonyl)isopropoxyimino]-2-(2-sulfoaminothiazol-4-yl)acetyl, 2-[2-(t-Butoxycarbonyl)isopropoxyimino]-2 -(2-triphenyl-methylamino-thiazol-4-yl)acetyl, 2-(2-Chloracetamidothiazol-4-yl)-2-isopropoxyiminoacetyl, 2-Methoxyimino-2-(2-sulfoaminothiazol-4-yl)acetyl, 2-(2-Aminothiazol-4-yl)-2-(carboxymethoxyimino)acetyl, 2-(2-Mesylaminothiazol-4-yl)-2-isopropoxyiminoacetyl, 2-(2-Imino-3-mesyl-4-thiazolin-4-yl)-2-isopropoxyiminoacetyl, 2-(2-Aminothiazol-4-yl)-2-(carboxyisopropoxyimino)acetyl etc.

f) (Acylamino)arylacetylgruppen der allgemeinen Formel

$$R^{140}-CO-NH-CH-CO-$$
$$|$$
$$R^{111}$$

in der $R^{111}$ die obige Bedeutung hat und $R^{140}$ eine Gruppe der allgemeinen Formel

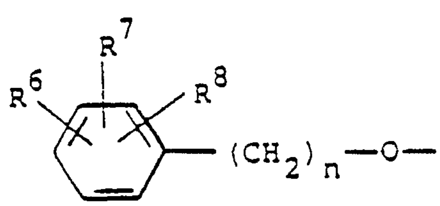

(worin $R^6$, $R^7$, $R^8$ und n die obige Bedeutung haben), Wasserstoff, niederes Alkyl, substituiertes niederes Alkyl, Amino, niederes Alkylamino, Cyanalkylamino oder Acylamino darstellt.

Bevorzugte (Acylamino)arylacetylgruppen der obigen Formel sind diejenigen, worin $R^{140}$ Amino oder Acylamino darstellt. Ebenfalls bevorzugt sind diejenigen Gruppen, worin $R^{111}$ Phenyl oder 2-Thienyl darstellt.

g) [[[3-Substituierte-2-oxo -1-imidazolidinyl]carbonyl]amino]arylacetylgruppen der allgemeinen Formel

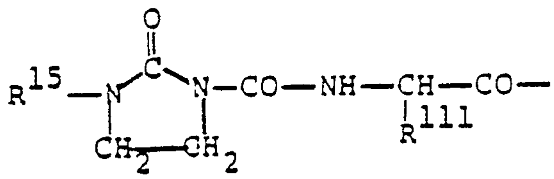

in der $R^{111}$ die oben gegebene Bedeutung hat, und $R^{15}$ Wasserstoff, niederes Alkylsulfonyl, -N = CH-$R^{111}$ (worin $R^{111}$ die oben gegebene Bedeutung hat), $R^{16}$-CO- (worin $R^{16}$ Wasserstoff oder gegebenenfalls durch Halogen substituiertes niederes Alkyl darstellt), eine wie unter $R^{111}$ oben definierte aromatische Gruppe oder gegebenenfalls durch einen oder mehrere der Reste Halogen, Cyan, Nitro, Amino und/oder Mercapto substituiertes niederes Alkyl darstellt.

Bevorzugte [[[3-substituierte-2-oxo -1-imidazolidinyl]carbonyl]amino]arylacetylgruppen der obigen Formel sind diejenigen, worin $R^{111}$ Phenyl oder 2-Thienyl darstellt. Ebenfalls bevorzugt sind diejenigen Gruppen, worin $R^{15}$ Wasserstoff, Methylsulfonyl, Phenylmethylamino oder 2-Furylmethylenamino darstellt.

In einer bevorzugten Ausführungsform des Chinolonyl- bzw. Azachinolonylsubstituenten in der 3-Stellung ist Z $R^{30}$-C, worin $R^{30}$ Wasserstoff, Chlor oder Fluor, vorzugsweise Wasserstoff oder Fluor, darstellt;

$R^{31}$ ist vorzugsweise niederes Alkyl, bevorzugt Aethyl, oder Halogen-niederes Alkyl, insbesondere Fluoräthyl, oder $C_3$-$C_7$-Cycloalkyl, vorzugsweise Cyclopropyl;

$R^{32}$ ist vorzugsweise niederes Alkyl, insbesondere Methyl, oder Piperazinyl, welches am Stickstoffatom in 4-Stellung durch niederes Alkyl, vorzugsweise durch Methyl substituiert sein kann;

$R^{33}$ ist vorzugsweise Wasserstoff, Chlor oder Fluor, insbesondere Wasserstoff oder Fluor, ganz besonders Fluor.

Der Chinolonyl- bzw. Azachinolonylsubstituent in 3-Stellung schliesst unter anderem Reste der folgenden Formeln ein:

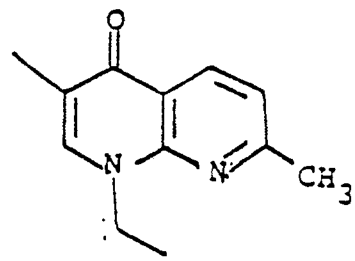

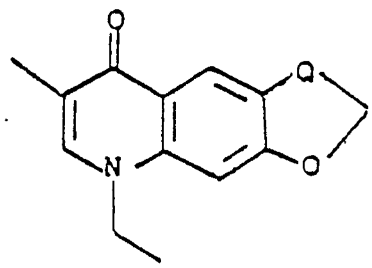

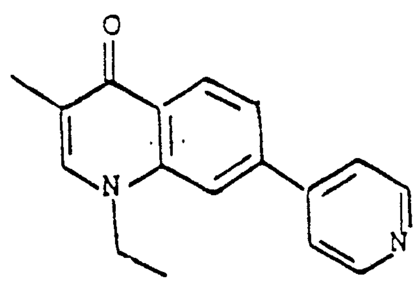

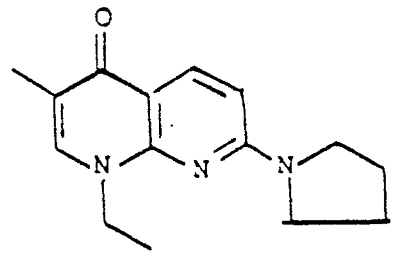

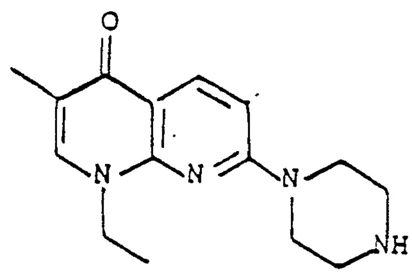

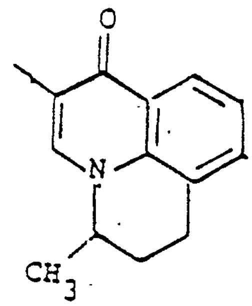

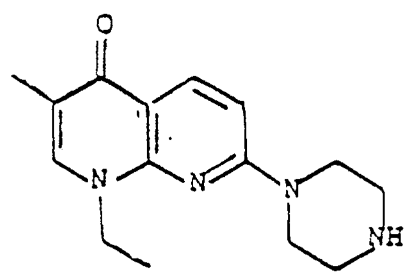

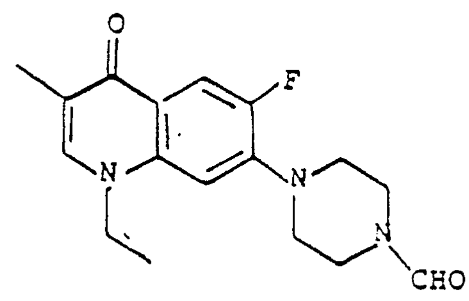

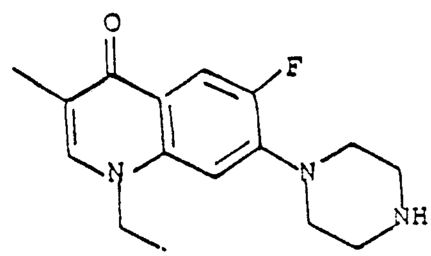

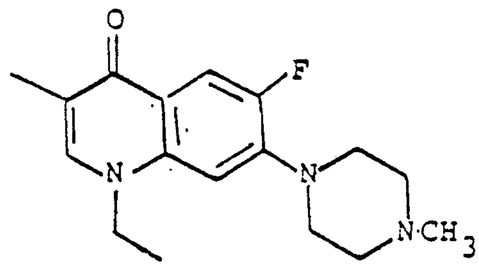

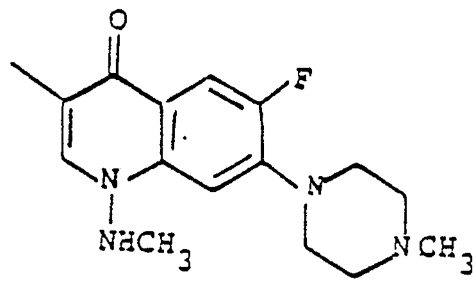

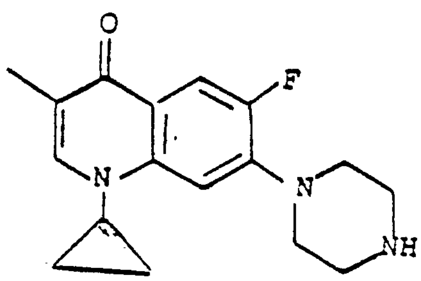

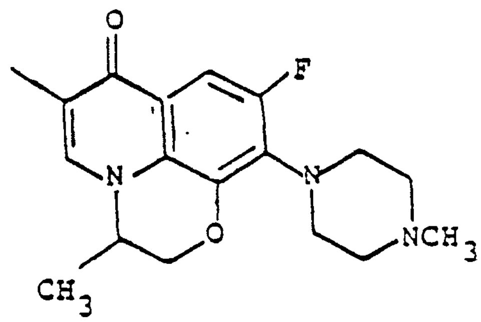

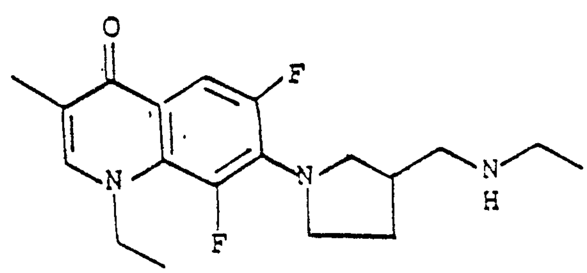

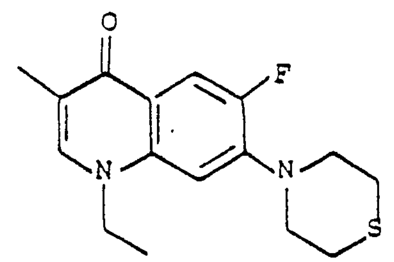

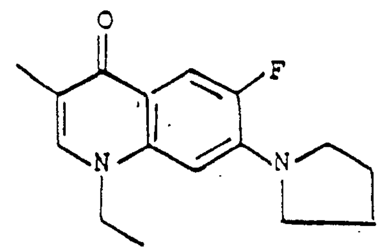

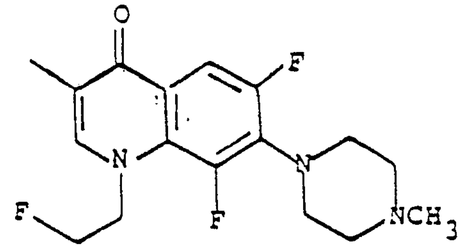

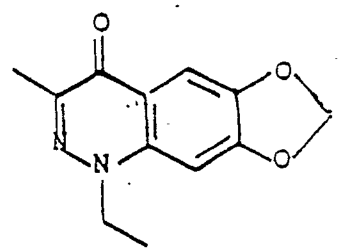

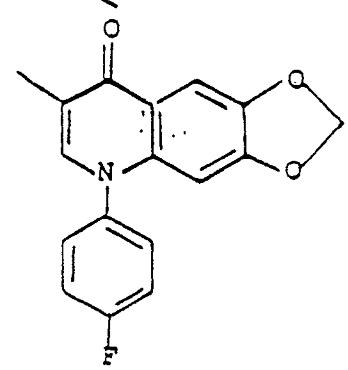

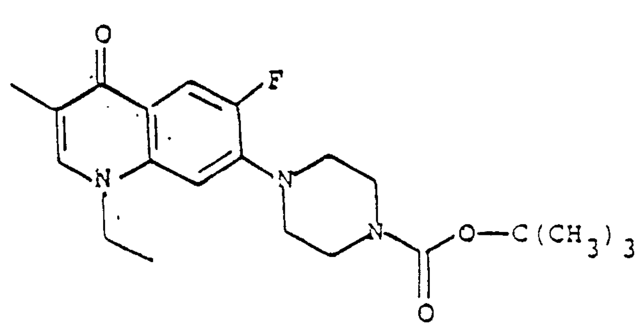

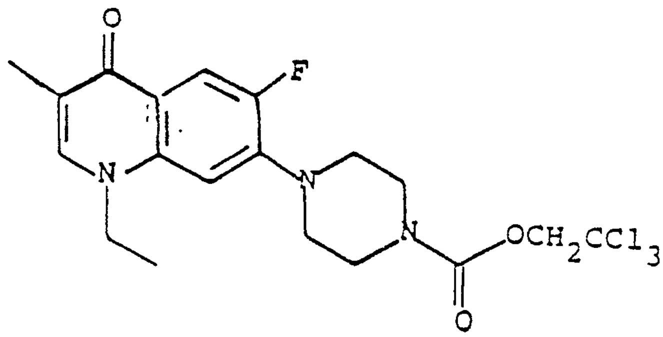

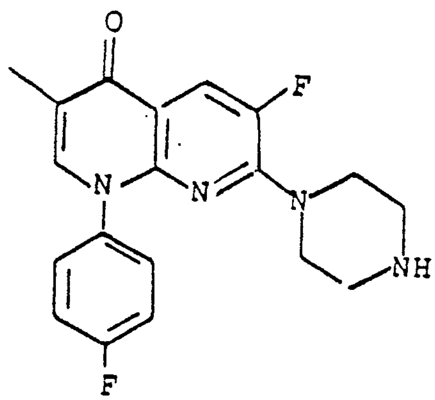

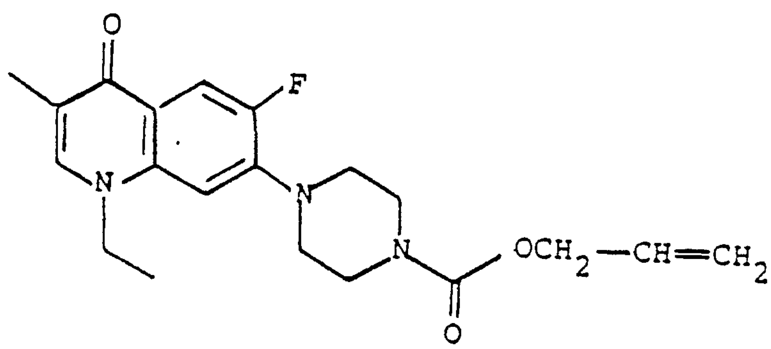

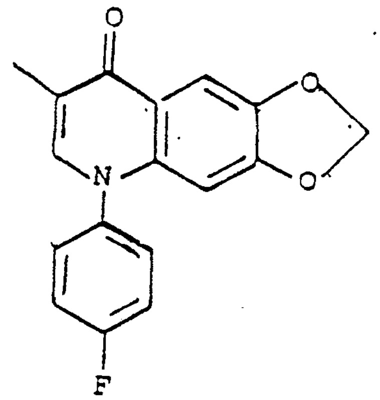

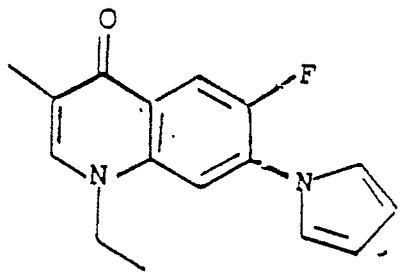

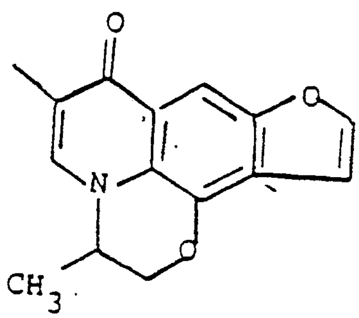

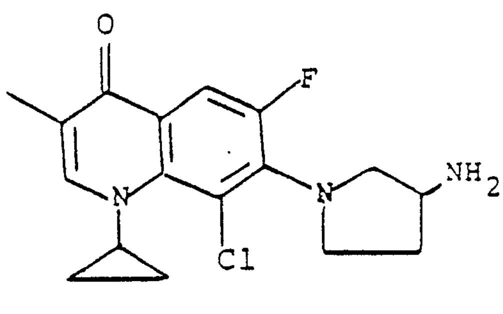

Eine bevorzugte Klasse der erfindungsgemässen Verbindungen ist diejenige der allgemeinen Formel

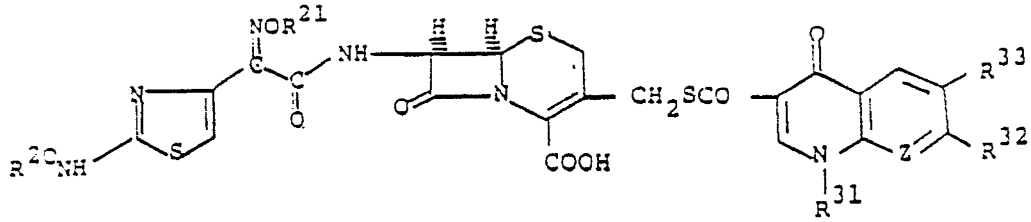

Ib

in der $R^1$ die oben gegebene Bedeutung hat, $R^{20}$ eine Aminoschutzgruppe, z.B. Trityl oder Chloracetyl, oder, vorzugsweise, Wasserstoff und $R^{21}$ Wasserstoff, niederes Alkyl oder eine Gruppe der allgemeinen Formel

$R^{22}$
|
-C-COOH
|
$R^{23}$ darstellt,

worin $R^{22}$ und $R^{23}$ Wasserstoff oder niederes Alkyl oder $R^{22}$ und $R^{23}$ zusammen mit dem Kohlenstoffatom, an das sie gebunden sind, einen 3-7-gliedrigen carbocyclischen Ring, z.B. Cyclopropyl, Cyclobutyl oder Cyclopentyl, darstellen.

Besonders bevorzugt sind Verbindungen der Formel Ib, worin $R^{20}$ Wasserstoff und $R^{21}$ Methyl oder eine Gruppe der Formel

$R^{22}$
|
-C-COOH
|
$R^{23}$

und $R^{22}$ und $R^{23}$ Wasserstoff oder Methyl darstellen.

Bevorzugt liegen die Gruppen

N-O-$R^{130}$ N-O-$R^{21}$
|| ||
-C- -C-

in der syn-Form, d.h. in der Z-Form, oder als Mischungen vor, in welchen die syn-Form überwiegt.

Als leicht hydrolysierbare Ester der Verbindungen der Formel I sind Verbindungen der Formel I zu verstehen, deren Carboxygruppe bzw. Carboxygruppen in Form einer leicht hydrolysierbaren Estergruppe vorliegt vorliegen. Beispiele solcher Ester, die herkömmlicher Art sein können, sind die niederen Alkanoyloxyalkylester, z.B. der Acetoxymethyl-, Pivaloyloxymethyl-, 1-Acetoxyäthyl- und der 1-Pivaloyloxyäthylester; die niederen Alkoxycarbonyloxyalkylester, z.B. der Methoxycarbonyloxymethyl-, 1-Aethoxycarbonyloxyäthyl- und der 1-Isopropoxycarbonyloxyäthylester; die Lactonylester, z.B. der Phthylidyl und der Thiophthalidylester; die niederen Alkoxymethylester, z.B. der Methoxymethylester und die niederen Alkanoylaminomethylester, z.B. der Acetamidomethylester. Auch andere Ester, z.B. die Benzyl- und Cyanmethylester können brauchbar sein.

Beispiele von Salzen der Verbindungen der Formel I sind Alkalimetallsalze, wie das Natrium- und das Kaliumsalz, Ammoniumsalze; Erdalkalimetallsalze, wie das Calciumsalz; Salze mit organischen Basen, wie die Salze mit Aminen, z.B. Salze mit N-Aethylpiperidin, Procain, Dibenzylamin, N,N′-Dibenzyläthylendiamin, Alkylaminen oder Dialkylaminen, sowie Salze mit Aminosäuren, wie z.B. Salze mit Arginin oder Lysin.

Die Verbindungen der Formel I, sofern sie eine basische funktionelle Gruppe, wie z.B. eine Aminogruppe besitzen, bilden ebenfalls Additionssalze mit organischen oder anorganischen Säuren. Beispiele für solche Salze sind Hydrohalogenide, beispielsweise Hydrochloride, Hydrobromide, Hydrojodide, sowie andere Mineralsäuresalze, wie Sulfate, Nitrate, Phosphate und dergleichen, Alkyl- und Monoarylsulfonate, wie Aethansulfonate, Toluolsulfonate, Benzolsulfonate und dergleichen und auch andere organische Säuresalze wie Acetate, Tartrate, Maleate, Citrate, Benzoate, Salicylate, Ascorbate und dergleichen.

Die Verbindungen der Formel I einschliesslich deren Salze und leicht hydrolysierbare Ester können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die obigen Acylderivate werden erfindungsgemäss dadurch hergestellt, dass man

(a) zur Herstellung eines leicht hydrolysierbaren Esters einer Carbonsäure der Formel I, eine Verbindung der allgemeinen Formel

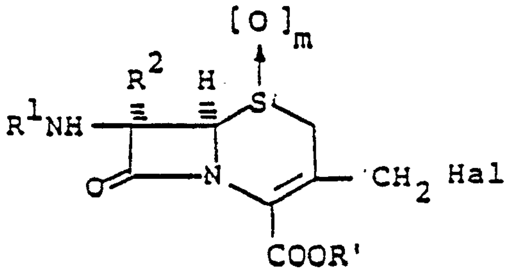

II

in der m, R' und $R^2$ obige Bedeutung haben, Hal Halogen und R′ den Rest eines leicht hydrolysierbaren Esters darstellen,
mit einem Salz einer Thiocarbonsäure der allgemeinen Formel

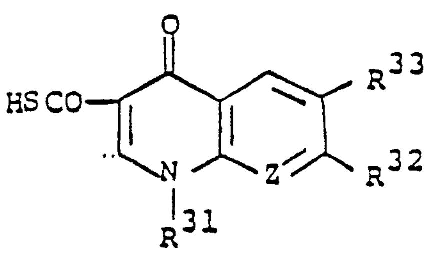

III

in der $R^{31}$, $R^{32}$ und $R^{33}$ obige Bedeutung haben,
umsetzt, oder dass man

(b) zur Herstellung einer Carbonsäure der Formel I, einen Ester der allgemeinen Formel

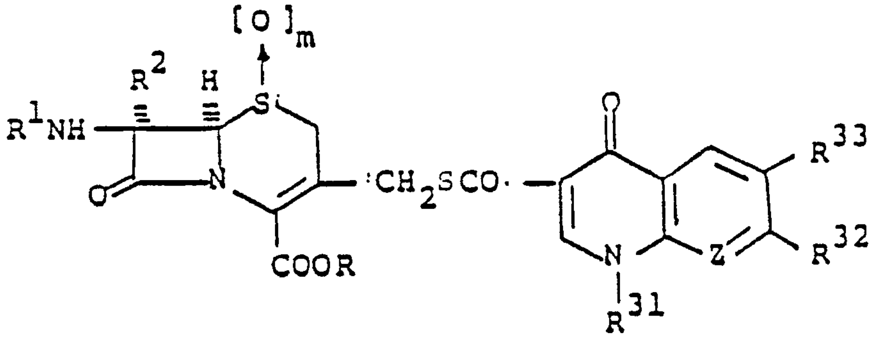

IV

in der m, $R^1$, $R^2$, $R^{31}$, $R^{32}$ und $R^{33}$ obige Bedeutung haben, und R eine Esterschutzgruppe darstellt, in die Carbonsäure der Formel I umwandelt, oder dass man

(c) zur Herstellung einer Verbindung der Formel I, worin m 0 darstellt, eine Verbindung der allgemeinen Formel

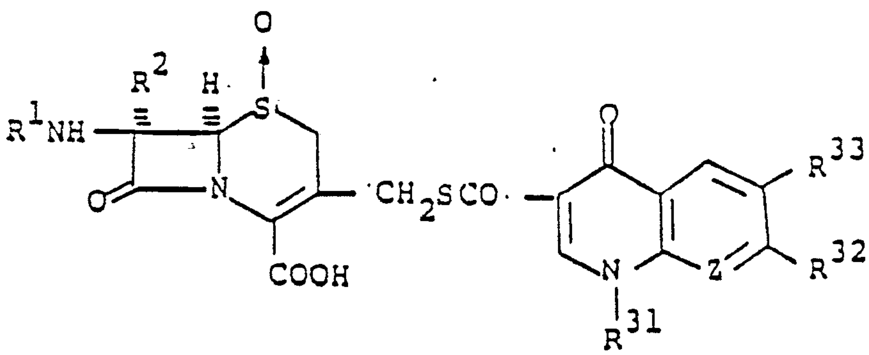

V

in der $R^1$, $R^2$, $R^{31}$, $R^{32}$ und $R^{33}$ obige Bedeutung haben, reduziert, oder dass man

(d) zur Herstellung einer Verbindung der Formel I, worin m 1 oder 2 darstellt, oder eines ihrer Ester oder Salze, eine Verbindung der allgemeinen Formel

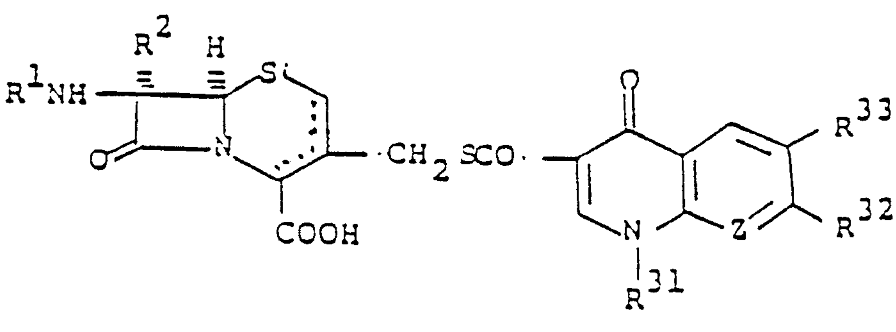

VI

in der $R^1$, $R^2$, $R^{31}$, $R^{32}$ und $R^{33}$ obige Bedeutung haben, und die gestrichelten Linien die Anwesenheit einer Δ2- oder Δ3-Doppelbindung anzeigen, oder eines ihrer Ester oder Salze oxidiert, oder dass man

(e) zur Herstellung einer Verbindung der Formel I, worin $R^1$ einen Aminosubstituenten enthält, oder eines ihrer Ester oder Salze, die Aminoschutzgruppe im Substituenten $R^{10}$ einer Verbindung der allgemeinen Formel

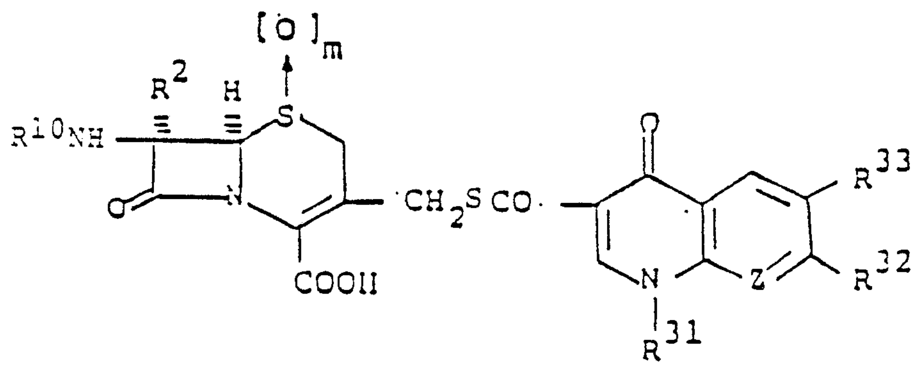

VII

in der m, $R^2$, $R^{31}$, $R^{32}$ und $R^{33}$ obige Bedeutung haben, und $R^{10}$ einen eine geschützte Aminogruppe enthaltenden Acylrest darstellt,

oder eines ihrer Ester oder Salze abspaltet, oder dass man

(f) zur Herstellung eines leicht hydrolysierbaren Esters einer Verbindung der Formel I, eine Carbonsäure der Formel I einer entsprechenden Veresterung unterwirft, oder dass man

(g) zur Herstellung von Salzen oder Hydraten einer Verbindung der Formel I bzw. von Hydraten besagter Salze, einer Verbindung der Formel I in ein Salz oder Hydrat bzw. in ein Hydrat des besagten Salzes überführt.

Die erfindungsgemässe Umsetzung des Halogenids der Formel II mit einem Salz der Carbonsäure der Formel III gemäss Variante a) des erfindungsgemässen Verfahrens wird vorzugsweise in einem nicht-hydroxylierten Lösungsmittel durchgeführt, wie z.B. in Dimethylformamid, Methylenchlorid oder N,N'-Dimethylacetamid. Geeignete Salze der Carbonsäure der Formel III sind z.B. die Natrium-, Kalium-, Cäsium-, t-Butylammonium- oder Tetramethylammoniumsalze. Hal in Formel II bedeutet Halogen, insbesondere Brom oder Jod. Diese Umsetzung wird bevorzugt bei etwa 0-80 °C, insbesondere bei Zimmertemperatur, durchgeführt.

Esterschutzgruppen R der in Variante b) des erfindungsgemässen Verfahrens verwendeten Ester der Formel IV sind vorzugsweise solche, welche unter milden Bedingungen in die freie Carboxygruppe umgewandelt werden können, z.B. t-Butyl, p-Nitrobenzyl, Benzhydryl, Allyl, etc. Auch die oben erläuterten Reste eines leicht hydrolysierbaren Esters können verwendet werden. Die Esterschutzgruppen werden z.B. wie folgt abgespalten: p-Nitrobenzyl durch Hydrolyse in Gegenwart von Natriumsulfid bei (oder unterhalb) 0 °C bis Zimmertemperatur in einem Lösungsmittel, wie z.B. Dimethylformamid (vorzugsweise wässrig); t-Butyl durch Umsetzung mit Trifluoressigsäure in Gegenwart von Anisol bei etwa 0 °C bis Zimmertemperatur, mit oder ohne zusätzliches Lösungsmittel, wie z.B. Methylenchlorid; Allyl durch Palladium-(O)-katalysierte Transallylierung in Gegenwart eines Natrium- oder Kaliumsalzes der 2-Aethylcapronsäure, siehe z.B. J. Org. Chem. 1982, 47, 587.

Die Reduktion der Sulfoxide der allgemeinen Formel V nach Variante c) des erfindungsgemässen Verfahrens kann nach einer Vielzahl von Reaktionen erfolgen, z.B. durch Behandlung mit Phosphortrichlorid in Dimethylformamid oder mit Trifluoressigsäureanhydrid in Gegenwart von Natriumjodid in Aceton/Methylenchlorid. Die Temperatur dieser Reaktionen liegt bevorzugt ei etwa 0 bis -20 °C, insbesondere bei 0 °C.

Durch die Oxidation der Verbindungen der Formel VI gemäss Variante d) des erfindungsgemässen Verfahrens wird ein allfälliges Δ2-Isomere der Formel VI in das entsprechende Δ3 Isomere der Formel I, worin m 1 oder 2 darstellt, übergeführt. Diese Oxidation wird durch Behandeln mit einem organischen oder anorganischen Oxidationsmittel durchgeführt. Als Oxidationsmittel dienen verschiedene, Sauerstoff leicht abgebende Verbindungen, wie z.B. organische Peroxide, z.B. monosubstituierte organische Peroxide wie $C_1$-$C_4$-Alkyl- oder Alkanoylhydroperoxide, wie t-Butylhydroperoxid, Perameisensäure oder Peressigsäure; sowie phenylsubstituierte Derivate dieser Hydroperoxide, wie Cumolhydroperoxid oder Perbenzoesäure. Der Phenylsubstituent kann gegebenenfalls eine weitere niedere Gruppe, z.B. $C_1$-$C_4$-Alkyl oder $C_1$-$C_4$-Alkoxy oder auch Halogen oder eine Carboxygruppe tragen, z.B. 4-Methylperbenzoesäure, 4-Methoxyperbenzoesäure, 3-Chlorperbenzoesäure oder Monoperphthalsäure. Als Oxidationsmittel können auch verschiedene anorganische Oxidationsmittel verwendet werden, z.B. Wasserstoffperoxid; Oxon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen, inerten Lösungsmittel, wie Tetrahydrofuran, Dioxan, Methylenchlorid, Chloroform, Aethylacetat oder Aceton; oder in einem protischen Lösungsmittel, wie Wasser, einem niederen Alkanol, z.B. Methanol oder Aethanol, oder einer niederen, gegebenenfalls halogenierten Alkancarbonsäure, z.B. Ameisensäure, Essigsäure oder Trifluoressigsäure. Die Reaktionstemperatur bewegt sich vornehmlich im Bereiche von -20° C bis +50C.

Bei Verwendung von äquimolaren Mengen Oxidationsmittel bzw. leichtem Ueberschuss im Verhältnis zum Ausgangsmaterial erhält man vornehmlich das entsprechende Sulfoxid, d.h. eine Verbindung der Formel I, worin m die Zahl 1 darstellt. Erhöht man die Menge des Oxidationsmittels auf das Doppelte des stöchiometrischen Verhältnisses oder mehr, bildet sich das entsprechende Sulfon, d.h. eine Verbindung der Formel I, worin m die Zahl 2 darstellt. Es ist ebenfalls möglich, das Sulfon der Formel I aus dem entsprechenden Sulfoxid durch Behandlung mit dem Oxidationsmittel in äquimolarer oder grösserer Menge zu erhalten. Die Verfahrensbedingungen sind im wesentlichen die gleichen wie bie der Herstellung des Sulfoxids.

Durch Abspaltung der Aminoschutzgruppe im Substituenten $R^{10}$ einer Verbindung der Formel VII gemäss Variante e) des erfindungsgemässen Verfahrens erhält man entsprechende Verbindungen der Formel I mit einer freien Aminogruppe. Mögliche Aminoschutzgruppen sind diejenigen, welche in der Peptidchemie verwendet werden, z.B. Alkoxycarbonylgruppen, z.B. t-Butoxycarbonyl etc., substituierte Alkoxycarbonylgruppen, z.B. Trichloräthoxycarbonyl etc., substituierte Aralkoxycarbonylgruppen, z.B. p-Nitrobenzyloxycarbonyl, Aralkylgruppen, z.B. Trityl oder Benzhydryl, oder Halogenalkanoylgruppen, wie z.B. Chloracetyl, Bromacetyl, Jodacetyl oder Trifluoracetyl.

Bevorzugte Aminoschutzgruppen sind t-Butoxycarbonyl (t-BOC) und Trityl.

Die Aminoschutzgruppen sind beispielsweise durch saure Hydrolyse abspaltbar (z.B. t-Butoxycarbonyl oder Trityl) oder auch durch basische Hydrolyse abspaltbar (z.B. Trifluoracetyl). Die Chlor, Brom- und Jodacetylgruppen können durch Behandeln mit Thioharnstoff abgespalten werden.

Durch saure Hydrolyse abspaltbare Schutzgruppen werden vorzugsweise mit Hilfe einer niederen Alkancarbonsäure, die gegebenenfalls halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperaturen angewendet werden können, z.B. im Bereiche von etwa 0° C bis +40° C. Alkalisch abspaltbare Schutzgruppen werden im allgemeinen mit verdünnter wässriger Lauge bei 0° C bis +30° C hydrolysiert. Die Chlor-, Brom- und Jodacetylschutzgruppen können mittels Thioharnstoff in saurem, neutralem oder alkalischem Milieu bei etwa 0° C bis +30° C abgespalten werden.

Zur Herstellung der leicht hydrolysierbaren Ester der Carbonsäuren der Formel I gemäss Variante f) des erfindungsgemässen Verfahrens wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin, beschleunigt werden. Die Veresterungsreaktion wird vorzugsweise in einem inerten organischen Lösungsmittel, wie Dimethylacetamid, Hexamethylphosphorsäuretriamid, Dimethylsulfoxid oder, vorzugsweise, Dimethylformamid durchgeführt. Die Temperatur liegt vorzugsweise im Bereiche von etwa 0-40° C.

Die Herstellung der Salze und Hydrate der Verbindungen der Formel I bzw. der Hydrate dieser Salze gemäss Variante g) des erfindungsgemässen Verfahrens kann in an sich bekannter Weise erfolgen, z.B. durch Umsetzen der Carbonsäure der Formel I mit einer äquimolaren menge der gewünschten Base, zweckmässig in einem Lösungsmittel, wie Wasser, oder in einem organischen Lösungsmittel, wie Aethanol, Methanol, Aceton und dergleichen. Die Temperatur der Salzbildung ist nicht kritisch, sie liegt im allgemeinen bei Raumtemperatur kann aber auch leicht darüber oder darunter, etwa im Bereiche von 0° C bis +50° C, liegen.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I oder eines ihrer Salze) einer feuchten Atmosphäre, z.B. bei etwa +10° C bis +40° C, ausgesetzt werden.

Das folgende Reaktionsschema I veranschaulicht das Verfahren zur Herstellung der erfindungsgemässen Produkte:

Schema I

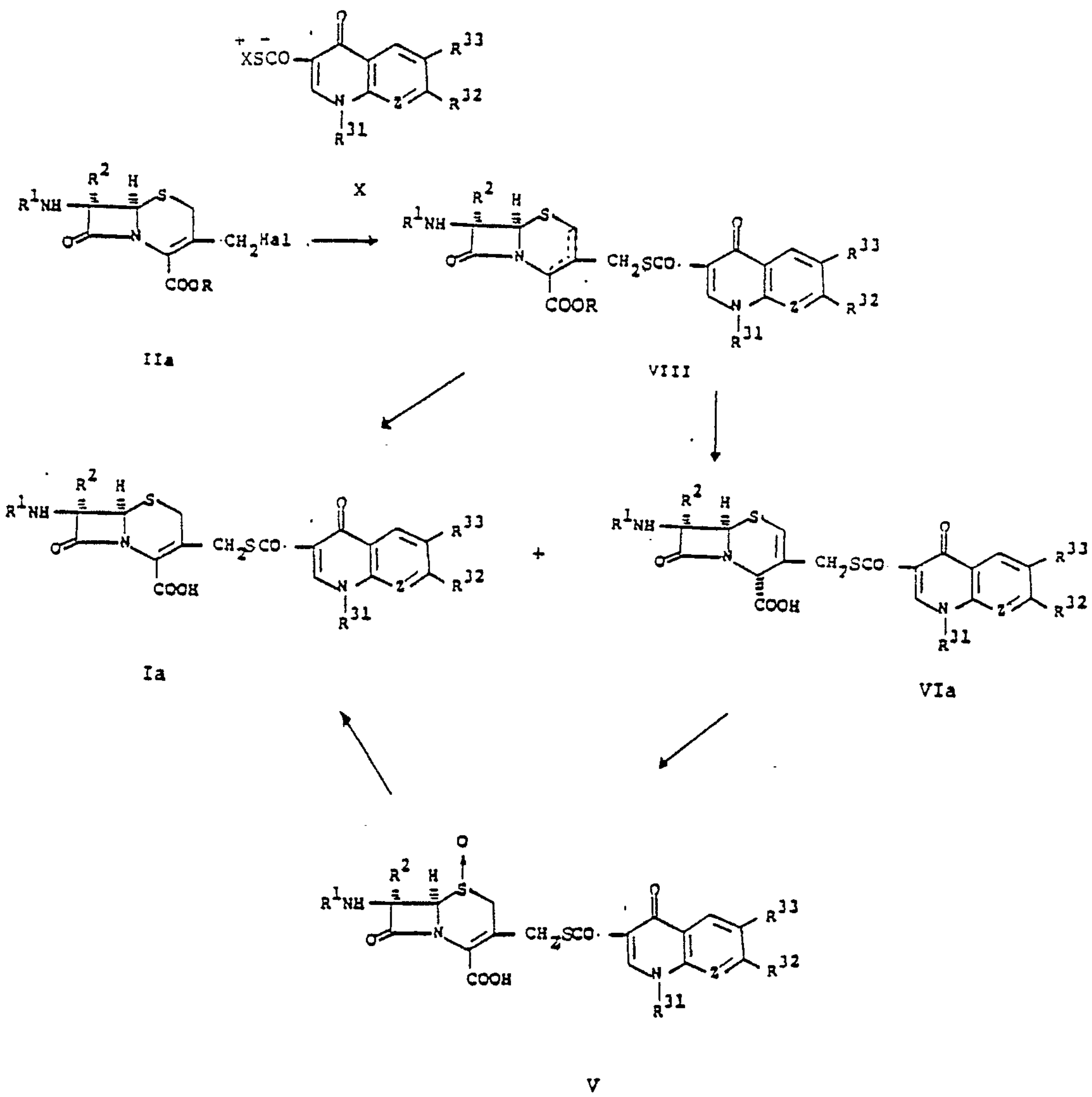

X

IIa

VIII

Ia

+

VIa

V

$R^1$, $R^2$, $R^{31}$, $R^{32}$, $R^{33}$, R, Z und die gestrichelen Bindungen haben alle die oben gegebene Bedeutung, und X stellt ein Kation dar.

Erläuterungen zum Schema I:

Die Carbonsäureform des entsprechenden Chinolons wird durch Behandeln mit einem 2-Fluor-1-methylpyridiniumsalz aktiviert. Durch Behandeln dieser aktivierten Säure mit Triphenylmethylmercaptan und 4-Dimethylaminopyridin erhält man einen Thioester der Formel

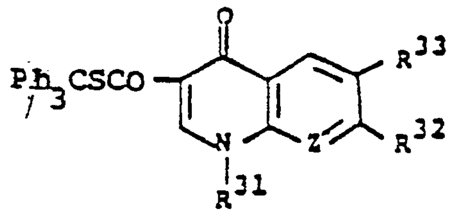

IX

in der $R^{31}$, $R^{32}$, $R^{33}$ und Z die obige Bedeutung haben und Ph Phenyl darstellt.

Die Verbindung der Formel IX wird mit einer wässrigen Säure, vorzugsweise wässriger Salzsäure, behandelt und anschliessend mit einer Base, insbesondere Kaliumhydroxid, neutralisiert; man erhält ein Thiocarbonsäuresalz der Formel

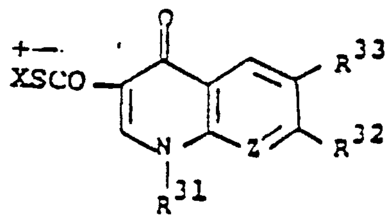

X

in der $R^{31}$, $R^{32}$, $R^{33}$ und Z die oben gegebene Bedeutung haben und $X^{+}$ ein Kation darstellt.

IIa → VIII

Die Verbindung der Formel IIa ist eine bekannte Verbindung oder ist in analoger Weise erhältlich (siehe z.B. die U.S. Patentschriften Nr. 4,406,899 und 4,266,049). Sie wird mit einem Salz des gewählten Chinolons der Formel III umgesetzt. Die Umsetzung erfolgt wie oben für die Verfahrens alternative a) beschrieben. Je nach Wahl der Esterschutzgruppe R und des Halogens Hal erhält man in Bezug auf die Doppelbindung im Cephemring ein Δ3- oder Δ2-Isomeres der Formel VIII.

VIII → Ia oder VIa

Die Esterschutzgruppe R der Verbindung der Formel VIII wird anschliessend abgespalten wie oben für die Verfahrensalternative b) beschrieben, wobei eine Carbonsäure der Formel Ia oder ein Gemisch davon mit dem Δ3-Isomer der Formel VIa erhalten wird.

VIa → V

Falls die Doppelbindung unter Bildung des Δ3-Isomeren isomerisiert wird, wird die so erhaltene Verbindung der Formel VIa, wie oben für Verfahrensalternative d) beschrieben, oxidiert, z.B. mit einer Persäure, wie z.B. 3-Chlorperbenzoesäure, in einem Lösungsmittel, wie z.B. Methylenchlorid, bei einer Reaktionstemperatur von etwa -20 °C bis 40 °C, vorzugsweise bei etwa 0 °C.

V → Ia

Die so erhaltene Verbindung der Formel V ist selbst ein unter Formel I fallendes Endprodukt. Sie kann jedoch zum Endprodukt Ia reduziert werden, wie oben für Verfahrensvariante c) beschrieben.

Verbindungen der Formel I mit den Gruppen

```
N-O-R¹³⁰                    N-O-R²¹
  ||                          ||
 -C-          oder           -C-          (vgl. oben)
```

liegen vorzugsweise als syn-Formen vor. Solche syn-Formen können erhalten werden durch Verwendung von Ausgangsmaterialien, worin diese syn-Form bereits vorliegt. Wahlweise kann ein erhaltenes syn anti-Gemisch einer Verbindung der Formel I in üblicher Weise in die entsprechenden syn- und anti-Formen aufgetrennt werden, beispielsweise durch Umkristallisation oder durch chromatographische Methoden unter Verwendung eines geeigneten Lösungsmittels bzw. Lösungsmittelgemisches.

Die Verbindungen der Formel I sowie die entsprechenden Salze bzw. die Hydrate dieser Produkte und Ester sind antibiotisch, insbesondere bakterizid wirksam. Sie können als Agenzien zur Bekämpfung von bakteriellen Infektionen (einschliesslich Harn- und Atemweginfektionen) bei Säugern, z.B. Hunden, Katzen, Pferden usw., sowie beim Menschen verwendet werden. Diese Cephalosporine sind wirksam gegen einen breiten Bereich sowohl gram-negativer als auch gram-positiver Bakterien.

Die in vitro Wirksamkeit der erfindungsgemässen Verbindung gegen eine Vielzahl gram-positiver und gram-negativer Mikroorganismen, ausgedrückt als Mindesthemmkonzentration in Mikrogramm ml und ermittelt unter Verwen dung der Reihenverdünnungsmethode (in Flüssigmedium) ist wie folgt:

Die in vitro Wirksamkeit von [6R-[6α,7β(Z)]]-7-[[(2-Amino-4-thiazolyl)(methoxyimino)acetyl]amino]-3-[[[-[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure-Mononatriumsalz-Trihydrat ist wie folgt:

| | MIC[a] | $ED_{50}$ [b] |
|---|---|---|
| Escherichia coli 257 | 0.0625 | <2 |
| DCO | 0.125 | |
| $DC_2$ | ≦0.0157 | |
| Pseudomonas aeruginosa 56 | 64 | |
| Staphylococcus aureus Smith | 2 | 43 |
| Streptococcus pneumoniae 6301 | ≦0.0157 | |

a) MIC in µg.ml
b) $ED_{50}$ in mg/kg

Zur Bekämpfung einer bakteriellen Infektion in einem Säuger kann eine erfindungsgemässe Verbindung mit einer Tagesdosis von etwa 5 bis etwa 500 mg/kg, vorzugsweise etwa 10-100 mg/kg, insbesondere etwa 10-55 mg/kg, behandelt werden.

Sämtliche Verabreichungsarten, welche bisher für die Penicillin- und Cephalosporintherapie in Frage kommen, sind ebenfalls für die erfindungsgemässen neuen Cephalosporine verwendbar. Verabreichungsarten sind demnach z.B. intravenöse, intramuskuläre und enterale Administrierungen.

Die erfindungsgemässen Produkte können als Heilmittel, z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial wie z.B. Wasser, Gelatine, Gummi arabicum, Milchzucker, Stärke, Magnesiumstearat, Talk, pflanzliche Oele, Polyalkylenglykole, Vaseline usw. enthalten. Die pharmazeutischen Präparate können in fester Form, z.B. als Tabletten, Dragées, Suppositorien, Kapseln oder in flüssiger Form, z.B. als Lösungen, Suspensionen oder Emulsionen vorliegen. Gegebenenfalls sind sie sterilisiert und bzw. oder enthalten Hilfsstoffe wie Konservierungs-, Stabilisierungs-, Netz- oder Emulgiermittel, Salze zur Veränderung des osmotischen Druckes, Anaesthetika oder Puffer. Sie können auch noch andere therapeutisch wertvolle Stoffe enthalten. Die Carbonsäuren der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien wie Wasser und isotonischer Kochsalzlösung, Lösungsmittelvermittler, wie Propylenglykol, zubereitet. Die leicht hydrolysierbaren Ester der Formel I kommen auch für die enterale Verabreichung in Betracht.

Beispiel 1

Eine Lösung von 2-Fluor-1-methylpyridinium-p-toluolsulfonat (21,25 g, 0,075 Mol) in trockenem Methylenchlorid (300 ml) wurde in einer Argonatmosphäre und in einem Eis-Salzbad auf -15 bis -10° C gekühlt. Dieses Gemisch wurde mit 22,16 g (0,06 Mol) 6,8-Difluor-1-(2-fluoräthyl)-1,4-dihydro-7-(4-methyl -1-piperazinyl)-4-oxo-3-chinolincarbonsäure und 7,32 g (0,06 Mol) 4-Dimethylaminopyridin langsam bei -10° C versetzt. Das erhaltene gelbe Reaktionsgemisch wurde 30 Minuten bei -10° C gerührt und anschliessend mit 16.6 g (0.06 Mol) Triphenylmethylmercaptan und 7,32 g (0,06 Mol) 4-Dimethylaminopyridin versetzt; das Gemisch wurde langsam auf 23° C gebracht und anschliessend 16 Stunden gerührt. Das Gemisch wurde anschliessend mit etwa 1 l Methylenchlorid verdünnt und unter verminderem Druck eingedampft. Es wurde 25 g eines gelben Oels erhalten, das zweimal an 100 g Kieselgel chromatographiert und mit 20% Aceton in Methylenchlorid eluiert wurde. Die das gewünschte Produkt enthaltenden Fraktionen wurden vereinigt und unter vermindertem Druck eingedampft. Es wurde so 6,8-Difluor1-(2-fluoräthyl)-1,4-dihydro-7-(4-methyl -1-piperazinyl)-4-oxo-3-chinolinthiocarbonsäure -S-triphenylmethylestersemihydrat erhalten.

Beispiel 2

Ein Gemisch von 6.1 g (9,6 mMol) 6,8-Difluor-1-(2-fluoräthyl)-1,4-dihydro-7-(4-methyl -1-piperazinyl)-4-oxo-3-chinolinthiocarbonsäure -S-triphenylmethylestersemihydrat und 8 ml wässriger 6N Salzsäure in 30 ml Tetrahydrofuran wurde in einer Argonatmosphäre 16 Stunden bei 23° C gerührt. Das Gemisch wurde bei 30° C eingedampft und anschliessend in 50 ml Wasser und 50 ml Chloroform aufgenommen. Dieses Gemisch wurde mit etwa 55 ml 1N wässriger Kaliumhydroxidlösung bis auf einen pH-Wert von etwa 11 behandelt. Nach zweimaliger Extraktion mit je 50 ml Chloroform und Extraktion der vereinigten Chloroformlösungen mit zweimal 50 ml Wasser wurde die organische Lösung über Magnesiumsulfat getrocknet und eingedampft. Es wurde 2,57 g eines Schaums erhalten, das gemäss Dünnschichtchromatogramm zur Hauptsache aus dem Ausgangsmaterial (Thioester) bestand. Die vereinigten wässrigen Phasen wurden durch 100 g einer $C_{18}$ Reverse Phase Chromatographie-Kolonne gegeben und mit einem Gradienten von 3% bis 20% Acetonitril-Wasser eluiert. Die das gewünschte Produkt enthaltenden Fraktionen wurden vereinigt und zur Entfernung des Acetonitrils unter vermindertem Druck eingedampft. Die wässrige Lösung wurde lyophilisiert, wobei 6,8-Difluor-1-(2-fluoräthyl)-1,4-dihydro-7-(4-methyl -1-piperazinyl)-4-oxo-3-chinolinthiocarbonsäure-kaliumsalz-hydrat als ein gelber Feststoff erhalten wurde.

Beispiel 3

Ein Gemisch von 1.8 g (4,07 mMol) 6,8-Difluor-1-(2-fluoräthyl)-1,4-dihydro-7-(4-methyl -1-piperazinyl)-4-oxo-3-chinolinthiocarbonsäure-kaliumsalz-hydrat und 2,0 g Propylenoxid in 20 ml trockenem Dimethylformamid wurde in einer Argonatmosphäre bei 23° C gerührt. Dieses Gemisch wurde mit einer Lösung von 3,69 g (4.5 mMol) [6R-[6α,7β(Z)]]-7-[[[[(Methoxyimino) -2-(triphenylmethyl)amino]-4-thiazolyl]acetyl]amino] -3-(jodmethyl)-8-oxo-5-thia-1-azabicyclo[4.2.0]oct-2-en -2-carbonsäure-t-butylester in 25 ml trockenem Methylenchlorid versetzt und das Ganze anschliessend 36 Minuten in einer Argonatmosphäre gerührt. Das Reaktionsgemisch wurde in 100 ml Aethylacetat aufgenommen, mit 100 ml Wasser und anschliessend zweimal mit je 100 ml wässriger Kochsalzlösung gewaschen. Die wässrigen Waschflüssigkeiten wurden zweimal mit je 100 ml Aethylacetat rückextrahiert. Die organischen Extrakte wurden vereinigt, über Magnesiumsulfat getrocknet und filtriert. Das Lösungsmittel wurde unter vermindertem Druck abgedampft, wonach 4,1 g eines Rohprodukts erhalten wurde, das an 125 g Kieselgel chromatographiert wurde. Eluieren mit 2% bis 4% Methanol in Chloroform lieferte Fraktionen 6-13 mit dem gewünschten Produkt. Nach Eindampfen zur Trockene unter vermindertem Druck erhielt man einen bräunlichen Feststoff: [6R-[6α,7β(Z)-]]-3-[[[[6.8-Difluor-1-(2-fluoräthyl)-1,4-dihydro-7-(4-methyl-1-piperazinyl)-4-oxo-3-chinolinyl]carbonyl]thio]-methyl]-7-[[[[(methoxyimino)-2-(triphenylmethyl)amino]-4-thiazolyl]acetyl]amino]-8-oxo-5-thia-1-azabicyclo-[4.2.0]oct-2-en-2-carbonsäure-1,1-dimethyläthylester.

Beispiel 4

Eine Lösung von 1,9 g (2.2 mMol) [6R-[6α.7β(Z)]]-3-[[[[6,8-Difluor-1-(2-fluoräthyl)-1,4-dihydro-7-(4-methyl-1-piperazinyl)-4-oxo-3-chinolinyl]carbonyl]thio]methyl]-7-[[[[(methoxyimino)-2-(triphenylmethyl)-amino]-4-thiazolyl]acetyl]amino]-8-oxo-5-thia-1-azabicyclo[4.2.0]oct-2-en-2-carbonsäure-1.1-dimethyläthylester, 2 ml Anisol und 0,1 ml 1,2-Aethandithiol in 20 ml Methylenchlorid wurde auf 0 °C abgekühlt und mit 20 ml kalter Trifluoressigsäure behandelt. Das Reaktionsgemisch wurde 17 Stunde bei -20 °C stehen gelassen und anschliessend bei etwa 4 °C und unter vermindertem Druck eingedampft. Der Rückstand wurde in 10 ml Methylenchlorid aufgenommen, anschliessend wurden 100 ml Aether schnell hinzugefügt. Der entstandene Niederschlag wurde abfiltriert und mit Aether gewaschen, wobei 2,03 g eines Feststoffes erhalten wurde. Dieses Material wurde in 20 ml Acetonitril-Wasser (1:1) gelöst und mit 5% wässriger Natriumbicarbonatlösung behandelt. Es wurde dabei 5 ml Dimethylformamid hinzugefügt, um entstandenes, gummiartiges Produkt zu lösen. Nun wurde 5%ige wässrige Natriumbicarbonatlösung bis zu pH 9,0 zugesetzt. Die wässrige Lösung wurde anschliessend an einer Reverse Phase Chromatographie-Kolonne ($C_{18}$) und mit 5% bis 60% Acetonitril-Wasser eluiert. Fraktionen 11-16 wurden mit 20% bis 25% Acetonitril in Wasser eluiert, vereinigt, unter vermindertem Druck eingedampft und anschliessend lyophilisiert. Man erhielt so das gewünschte Produkt als ein weisses Pulver: [6R-[6α,7β(Z)]]-7-[[2-Amino-4-thiazolyl)(methoxyimino)acetyl]amino]-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure-mononatriumsalz-trihydrat.

Die folgenden Verbindungen können durch die in den Beispielen veranschaulichten Methoden hergestellt werden:

[6R-[6α,7β]-3-[[[[6,8-Difluor-1-(2-fluoräthyl)-1,4-dihydro-7-(4-methyl-1-piperazinyl)-4-oxo-3-chinolinyl]-carbonyl]thio]methyl]-8-oxo-7-[(phenoxyacetyl)amino]-5-thia-1-azabicyclo[4.2.0]oct-2-en-2-carbonsäure,

[6R-[6α,7β(Z)]]-7-[[2-Amino-4-thiazolyl)methoxyimino]acetyl]amino]-3-[[[[1-cyclopropyl-6-fluor-1,4-dihydro-4-oxo-7-(1-piperazinyl)-3-chinolinyl]carbonyl]thio]methyl]-8-oxo-5-thia-1-azabicyclo[4.2.0]oct-2-en-2-carbonsäure,

[6R-[6α,7β]-7-[(Cyanacetyl)amino]-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure,

[6R-[6α,7β]-7-[[[[(4-Aethyl-2,3-dioxo-1-piperazinyl)carbonyl]amino]phenylacetyl]amino]-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]-oct-2-en-2-carbonsäure,

[6R-[6α,7β(Z)]]-8-[[(2-Amino-4-thiazolyl)-[[(1-carboxy-1-methyl)äthoxy]imino]acetyl]amino]-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure,

[6R-[6α,7β(Z)]]-7-[[[(2-Amino-4-thiazolyl)(carboxymethoxy)imino]acetyl]amino]-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure,

[6R-[6α,7β(Z)]]-7-[[[(2-Amino-4-thiazolyl)methoxyimino]acetyl]amino]-3-[[[[9-fluor-3,7-dihydro-3-methyl-10-(4-methyl-1-piperazinyl)-7-oxo-2H-pyrido[1,2,3-de]-1,4-benzoxazin-6-yl]carbonyl]thio]methyl]-8-oxo-5-thia-1-azabicyclo[4.2.0]oct-2-en-2-carbonsäure,

[6R-[6α,7β)]-α-[[[2-Carboxy-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-7-yl]amino]carbonyl]-benzolessigsäure,

[6R-[6α,7β)]]-7-[[(2-Amino-4-thiazolyl)(methoxyimino)acetyl]amino]-3-[[[[6-fluor-1-(4-fluorphenyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure.

## Beispiel A

Herstellung von Trockenampullen für die intramuskuläre Verabreichung:

Es wird in üblicher Weise ein Lyophilisat von 1 g des [6R-[6α,7β(Z)]]-7-[[2-Amino-4-thiazolyl)-(methoxyimino)acetyl]amino]-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure-mononatriumsalz-trihydrats hergestellt und in eine Ampulle abgefüllt. Die sterile Wasserampulle enthält 10% Propylenglykol. Vor der Verabreichung wid das Lyophilisat mit 2,5 ml einer 2%-igen wässrigen Lidocain-hydrochloridlösung versetzt.

## Ansprüche

1. Acylderivate der allgemeinen Formel

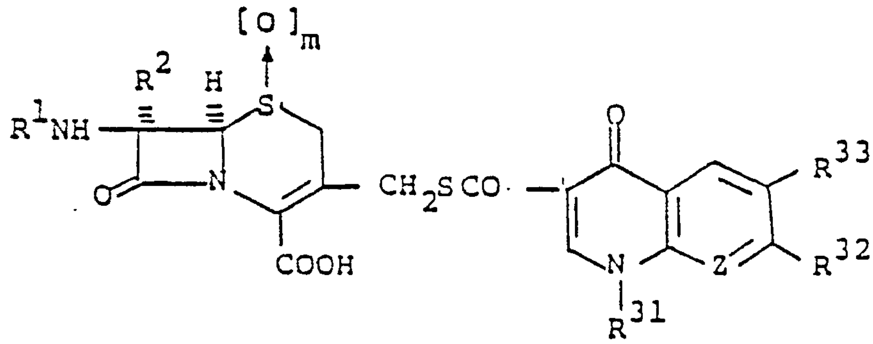

I

in der m 0, 1 oder 2, $R^1$ Wasserstoff oder eine Acylgruppe, $R^2$ Wasserstoff, niederes Alkoxy, niederes Alkylthio oder niederes Alkanoylamino, $R^{31}$ Wasserstoff, niederes Alkyl, niederes Alkenyl, $C_3$-$C_7$-Cycloalkyl, Halogen-niederes Alkyl oder Mono-, Di- oder Tri-halogenphenyl; Z $R^{30}$ -C oder Stickstoff, $R^{30}$ Wasserstoff oder Halogen, oder $R^{30}$ und $R^{31}$ zusammen eine $C_3$-$C_5$-Alkylengruppe, eine $C_2$-$C_4$-Alkylenmonooxygruppe oder eine $C_1$-$C_2$ Alkylendioxygruppe, $R^{32}$ Wasserstoff, Halogen, niederes Alkyl oder einen gegebenenfalls substituierten 5- oder 6-gliedrigen heterocyclischen Ring mit einem, zwei oder drei Sauerstoff-, Stickstoff- und/oder Schwefelatomen und $R^{33}$ Wasserstoff oder Halogen oder $R^{32}$ und $R^{33}$ zusammen eine $C_1$-$C_4$-Alkylendioxygruppe darstellen,
sowie leicht hydrolysierbare Ester und Salze dieser Verbindungen und Hydrate der Verbindungen der Formel I bzw. von deren Estern und Salzen.

2. Verbindungen nach Anspruch 1, dadurch gekennzeichnet, dass m 0 und $R^2$ Wasserstoff darstellt.

3. Verbindungen nach Anspruch 1 oder 2, dadurch gekennzeichnet, dass Z $R^{30}$-C darstellt, wobei $R^{30}$ Wasserstoff oder Halogen, $R^{31}$ niederes Alkyl, Halogen-niederes Alkyl oder $C_3$-$C_7$-Cycloalkyl, $R^{32}$ niederes Alkyl, Piperazinyl oder niederes Alkylpiperazinyl und $R^{33}$ Wasserstoff oder Halogen darstellen.

4. Verbindungen nach Anspruch 3, dadurch gekennzeichnet, dass $R^{30}$ Wasserstoff oder Fluor, $R^{31}$ Aethyl, Fluoräthyl oder Cyclopropyl, $R^{32}$ Piperazinyl oder 4-Methylpiperazinyl und $R^{33}$ Wasserstoff oder Fluor darstellen.

5. Verbindungen nach einem der Ansprüche 1-4, dadurch gekennzeichnet, dass $R^1$ eine der folgenden Acylgruppen darstellt:

(a) eine aliphatische Gruppe der Formel

$R^5$-CO-

in der $R^5$ niederes Alkyl, $C_3$-$C_7$-Cycloalkyl, niederes Alkoxy, niederes Alkenyl, $C_3$-$C_7$-Cycloalkenyl oder Cyclohexadienyl oder durch einen oder mehrere der Reste Halogen, Cyan, Nitro, Amino, Mercapto, niederes Alkylthio oder Cyanmethylthio substituiertes niederes Alkyl oder niederes Alkenyl darstellt;

(b) eine carbocyclische aromatische Gruppe der allgemeinen Formel

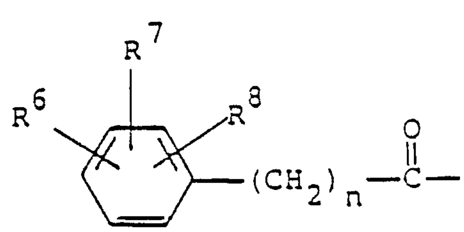

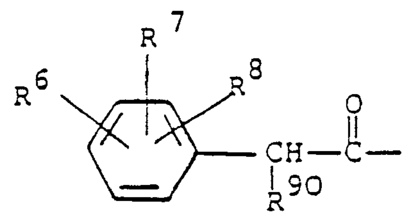

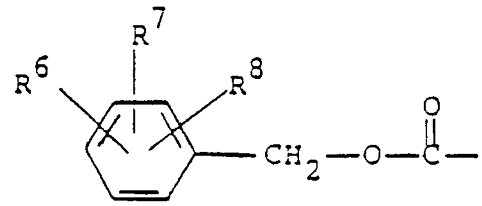

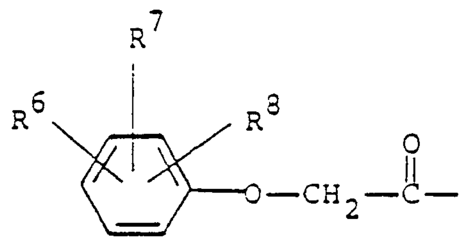

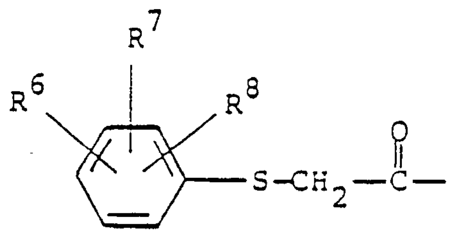

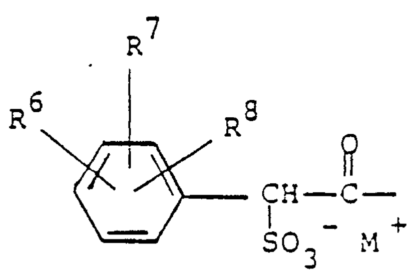

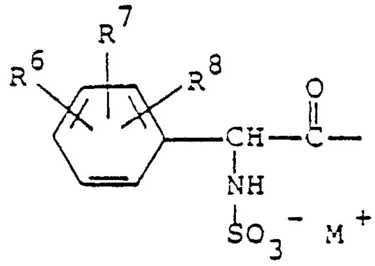

in der n 0, 1, 2 oder 3; $R^6$, $R^7$ und $R^8$ Wasserstoff, Halogen, Hydroxy, Nitro, Amino, Cyano, Trifluormethyl, $C_1$-$C_4$-Alkyl, $C_1$-$C_4$-Alkoxy oder Aminomethyl, $R^{90}$ Amino, Acylamino, Hydroxy, ein Carboxysalz, geschütztes Carboxy, Formyloxy oder Azido und M ein Kation darstellen;

(c) eine heteroaromatische Gruppe der allgemeinen Formeln

$R^{101}-(CH_2)_n-CO-$

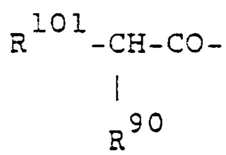

$R^{101}-O-CH_2-CO-$

$R^{101}-S-CH_2-CO-$ or

$R^{101}-CO-CO-$

in denen $R^{90}$ die oben gegebene Bedeutung hat, n 0, 1, 2 oder 3 und $R^{101}$ einen gegebenenfalls substituierten 5-, 6- oder 7-gliedrigen heterocyclischen Ring mit 1, 2, 3 oder 4 Stickstoff-, Sauerstoff- und oder Schwefelatomen darstellt;

(d) eine [[4-Substituierte-2,3-dioxo -1-piperazinyl)carbonyl]amino]arylacetylgruppe der Formel

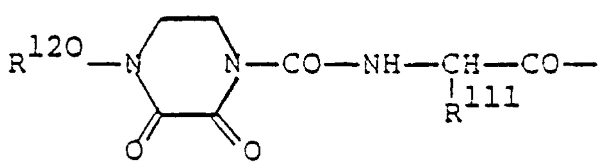

in der $R^{111}$ niederes Alkyl, Hydroxy-niederes Alkyl oder eine aromatische Gruppe der allgemeinen Formel

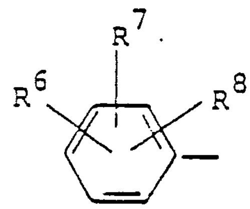

(in der $R^6$, $R^7$ und $R^8$ die oben gegebenen Bedeutung haben) oder einen wie für $R^{101}$ definierten heterocyclishen ring und $R^{120}$ gegebenenfalls durch einen oder mehrere der Reste Halogen, Cyan, Nitro, Amino und/oder Mercapto substituiertes niederes Alkyl darstellen;

(e) eine (substituierte Oxiimino)arylacetylgruppe der allgemeinen Formel

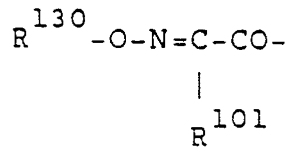

in der $R^{101}$ die oben gegebene Bedeutung hat und $R^{130}$ Wasserstoff, niederes Alkyl, $C_3$-$C_7$-Cycloalkyl, Carboxy-$C_3$-$C_7$-cycloalkyl oder substituiertes niederes Alkyl darstellt, wobei die niedere Alkylgruppe substituiert ist durch einen oder mehrere der Reste Halogen, Cyan, Nitro, Amino, Mercapto, niederes Alkylthio, eine durch $R^{111}$ oben definierte aromatische Gruppe, Carboxy (einschliesslich dessen Salze), niederes Alkanoylamino, niederes Alkoxycarbonyl, Phenylmethoxycarbonyl, Diphenylmethoxycarbonyl, Hydroxy-(niederes Alkoxy)phosphinyl, Dihydroxyphosphinyl, Hydroxy(phenylmethoxy)phosphinyl und/oder di-(niederes Alkoxy)phosphinyl;

(f) eine (Acylamino)arylacetylgruppe der allgemeinen Formel

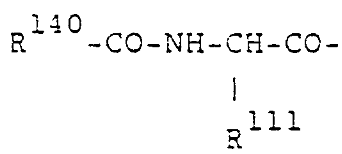

in der $R^{111}$ die oben gegebene Bedeutung hat und $R^{140}$ eine Gruppe der allgemeinen Formel

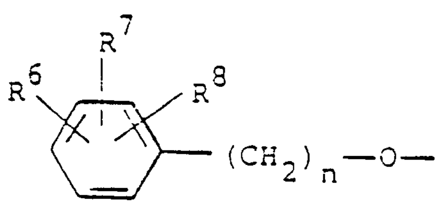

(worin $R^6$, $R^7$, $R^8$ und n die obige Bedeutung haben), Wasserstoff, niederes Alkyl, substituiertes niederes Alkyl, Amino, niederes Alkylamino, Cyanalkylamino oder Acylamino darstellt;

(g) eine [[[3-Substituierte-2-oxo -1-imidazolidinyl]carbonyl]amino]arylacetylgruppe der allgemeinen Formel

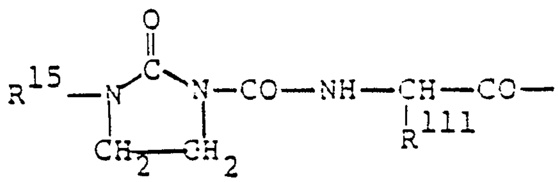

in der $R^{111}$ die oben gegebene Bedeutung hat, und $R^{15}$ Wasserstoff, niederes Alkylsulfonyl, $-N=CH-R^{111}$ (worin $R^{111}$ die oben gegebene Bedeutung hat), $R^{16}$ -CO(worin $R^{15}$ Wasserstoff oder gegebenenfalls durch Halogen substituiertes niederes Alkyl darstellt), eine wie unter $R^{111}$ oben definierte aromatische Gruppe oder gegebenenfalls durch einen oder mehrere der Reste Halogen, Cyan, Nitro, Amino und oder Mercapto substituiertes niederes Alkyl darstellt.

6. Verbindungen nach einem der Ansprüche 1-5, dadurch gekennzeichnet, dass R' eine carbocyclische aromatische Grupper der allgemeinen Formel

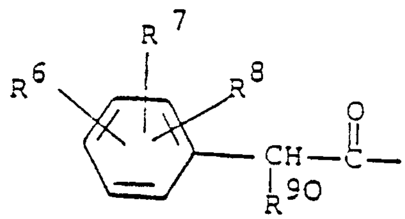

darstellt, in der $R^{90}$ Amino, Acylamino, Hydroxy, ein Carboxysalz, Benzyloxycarbonyl, Formyloxy oder Azido und $R^6$, $R^7$ und $R^8$ Wasserstoff, Halogen, Hydroxy, Nitro, Amino, Cyan, Trifluormethyl, $C_1$-$C_4$-Alkyl, $C_1$-$C_4$-Alkoxy oder Aminomethyl darstellen.

7. Verbindungen nach Anspruch 6, dadurch gekennzeichnet, dass $R^6$, $R^7$ und $R^8$ Wasserstoff und $R^{90}$ Wasserstoff oder Hydroxy darstellen.

8. Verbindungen nach einem der Ansprüche 1-5, dadurh gekennzeichnet, dass R' eine Acylgruppe der allgemeinen Formel

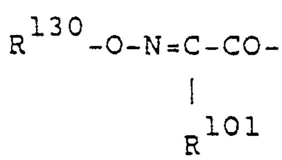

darstellt, in der $R^{101}$ eine gegebenenfalls durch Halogen, Hydroxy, Nitro, Amino, Cyan, Trifluormethyl, $C_1$-$C_4$-Alkyl oder $C_1$-$C_4$-Alkoxy substituierten 5-, 6- oder 7-gliedrigen heterocyclischen Ring mit 1, 2, 3 oder 4 Stickstoff-, Sauerstoff- und/oder Schwefelatomen und $R^{130}$ Wasserstoff, niederes Alkyl, $C_3$-$C_7$-Cycloalkyl, Carboxy-$C_3$-$C_7$-Cycloalkyl oder durch einen oder mehrere der Reste Halogen, Cyan, Nitro, Amino, Mercapto, niederes Alkylthio, eine durch $R^{11}$ gemäss Anspruch 5 definierte aromatische Gruppe, Carboxy (einschliesslich dessen Salze), niederes Alkanoylamino, niederes Alkoxycarbonyl, Phenylmethoxycarbonyl, Diphenylmethoxycarbonyl, Hydroxy(niederes Alkoxy)phosphinyl, Dihydroxyphosphinyl, Hydroxy(phenylmethoxy)phosphinyl und/oder Di(niederes Alkoxy)phosphinyl substituiertes niederes Alkyl bedeuten.

9. Verbindungen nach einem der Ansprüche 1-5 und 8, dadurch gekennzeichnet, dass R' eine Acylgruppe der allgemeinen Formel

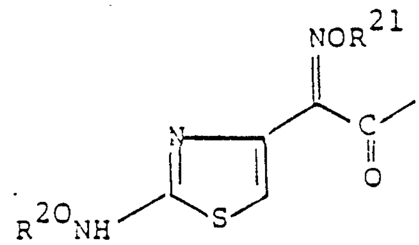

darstellt, in der $R^{20}$ Wasserstoff oder eine Aminoschutzgruppe und $R^{21}$ Wasserstoff, niederes Alkyl oder eine Gruppe der allgemeinen Formel

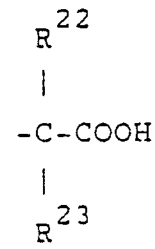

darstellen, worin $R^{22}$ und $R^{23}$ Wasserstoff, niederes Alkyl oder zusammen mit dem verbindenden Kohlenstoffatom einen 3-7-gliedrigen carbocyclischen Ring darstellen.

10. Verbindungen nach Anspruch 9, dadurch gekennzeichnet, dass $R^{20}$ Wasserstoff und $R^{21}$ Methyl oder

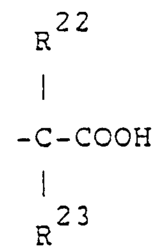

darstellen, worin $R^{22}$ und $R^{23}$ Wasserstoff oder Methyl bedeuten.

11. Verbindungen nach Anspruch 1 der allgemeinen Formel

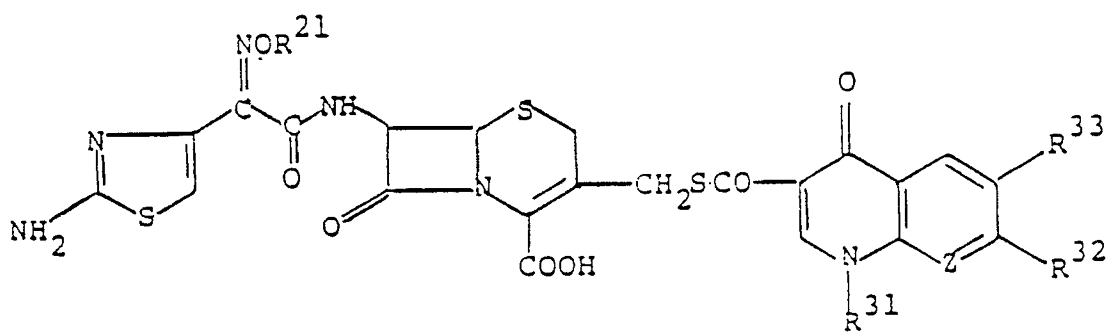

in der $R^{21}$ die in Anspruch 9 und Z, $R^{31}$, $R^{32}$ und $R^{33}$ die in Anspruch 1 gegebene Bedeutung haben.

12. Verbindungen nach einem der Ansprüche 1-11, dadurch gekennzeichnet, dass $R^{21}$ Methyl oder eine Gruppe der allgemeinen Formel

$$\begin{array}{c} R^{22} \\ | \\ -C-COOH \\ | \\ R^{23} \end{array}$$

$R^{22}$ und $R^{23}$ Wasserstoff oder Methyl, Z die Gruppe $R^{30}$-C, $R^{30}$ Wasserstoff oder Halogen, $R^{31}$ niederes Alkyl, Halogen-niederes Alkyl oder $C_3$-$C_7$-Cycloalkyl, $R^{32}$ niederes Alkyl, Piperazinyl oder niederes Alkylpiperazinyl und $R^{33}$ Wasserstoff oder Halogen darstellen.

13. Verbindungen nach Anspruch 12, dadurch gekennzeichnet, dass $R^{30}$ Wasserstoff oder Fluor, $R^{31}$ Aethyl, Fluoräthyl oder Cyclopropyl, $R^{32}$ Methyl, 4-Methyl-piperazinyl oder Piperazinyl und $R^{33}$ Wasserstoff oder Fluor darstellen.

14. Verbindungen nach Anspruch 1, gekennzeichnet als [6R-[6-alpha,7-beta(Z)]]-7-[[(2-amino -4-thiazolyl)(R$^{21}$-oxyimino)acetyl]amino] -3-[[[[6-R$^{33}$,8-R$^{30}$, 1-R$^{31}$-1,4-dihydro -7-(4-R$^{34}$-1-piperazinyl) -4-oxo-3-chinolinyl]carbonyl]thio]methyl] -8-oxo-5-thia-1-azabicyclo[4.2.0]oct -2-en-2-carbonsäuren und deren Salze und Hydrate dieser Carbonsäuren und Salze, worin $R^{33}$ und $R^{30}$ Wasserstoff oder Halogen, $R^{31}$ Wasserstoff, niederes Alkyl oder 2-Halogen-niederes Alkyl, $R^{34}$ Wasserstoff oder niederes Alkyl und $R^{21}$ Wasserstoff, niederes Alkyl oder eine Gruppe der allgemeinen Formel

$$\begin{array}{c} R^{22} \\ | \\ -C-COOH \\ | \\ R^{23} \end{array}$$

und $R^{22}$ und $R^{23}$ Wasserstoff oder niederes Alkyl bedeuten.

15. Verbindungen nach Anspruch 14, dadurch gekennzeichnet, dass $R^{21}$ Methyl darstellt.

16. Verbindungen nach Anspruch 15, dadurch gekennzeichnet, dass $R^{33}$ und $R^{30}$ Wasserstoff oder Fluor, $R^{31}$ Aethyl oder 2-Fluoräthyl und $R^{34}$ Methyl darstellen.

17. Verbindungen nach Anspruch 16, dadurch gekennzeichnet, dass $R^{33}$ und $R^{30}$ Fluor und $R^{31}$ 2-Fluoräthyl darstellen.

18. Verbindungen nach Anspruch 15, dadurch gekennzeichnet, dass $R^{33}$ Fluor, $R^{30}$ Wasserstoff, $R^{31}$ 2-Fluoräthyl und $R^{34}$ Methyl darstellen.

19. Verbindungen nach Anspruch 15, dadurch gekennzeichnet, dass $R^{33}$ Fluor, $R^{30}$ Wasserstoff, $R^{31}$ Aethyl und $R^{34}$ Methyl darstellen.

20. Verbindungen nach Anspruch 15, dadurch gekennzeichnet, dass $R^{33}$ Fluor, $R^{31}$ 2-Fluoräthyl, $R^{30}$ Wasserstoff und $R^{34}$ Methyl darstellen.

21. Verbindungen nach einem der Ansprüche 8-20, dadurch gekennzeichnet, dass die Gruppen der allgemeinen Formeln

$$\begin{array}{c} N-O-R^{130} \\ \| \\ -C- \end{array} \quad \text{und} \quad \begin{array}{c} N-O-R^{21} \\ \| \\ -C- \end{array}$$

in der synisomeren Form oder als Gemische vorliegen, in denen die synisomere Form überwiegt.

22. Verbindungen nach Anspruch 1, gekennzeichnet als [6R-[6α.7β(Z)]]-7-[[2-Amino-4-thiazolyl)-(methoxyimino)acetyl]amino]-3-[[[[6,8-difluor-1-(2-fluoräthyl)-1,4-dihydro-7-(4-methyl-1-piperazinyl)-4-oxo-3-chinolinyl]carbonyl]thio] methyl]-8-oxo-5-thia-1-azabicyclo[4.2.0]oct-2-en-2-carbonsäure sowie Salze dieser Verbindung und Hydrate dieser Verbindung bzw. Salze.

23. Verbindungen gemäss einem der Ansprüche 1-22 als pharmazeutische Wirkstoffe.

24. Verbindungen gemäss einem der Ansprüche 1-22 als pharmazeutische Wirkstoffe zur Behandlung und Prophylaxe von Infektionskrankheiten.

25. Pharmazeutische Präparate, gekennzeichnet durch einen Gehalt an einer Verbindung gemäss einem der Ansprüche 1-22.

26. Pharmazeutische Präparate zur Behandlung und Prophylaxe von Infektionskrankheiten, gekennzeichnet durch einen Gehalt an einer Verbindung gemäss einem der Ansprüche 1-22.

27. Verfahren zur Herstellung der Verbindungen gemäss einem der Ansprüche 1-22, dadurch gekennzeichnet, das man

(a) zur Herstellung eines leicht hydrolysierbaren Esters einer Carbonsäure der Formel I, eine Verbindung der allgemeinen Formel

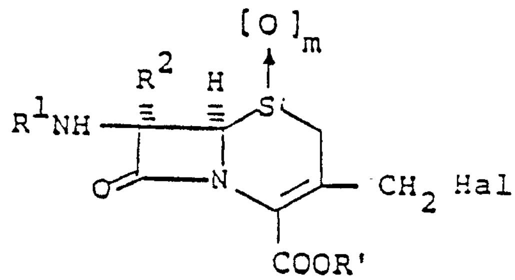

II

in der m, $R^1$ und $R^2$ die in Anspruch 1 gegebene Bedeutung haben, Hal Halogen und $R'$ den Rest eines leicht hydrolysierbaren Esters darstellen,
mit einem Salz einer Thiocarbonsäure der allgemeinen Formel

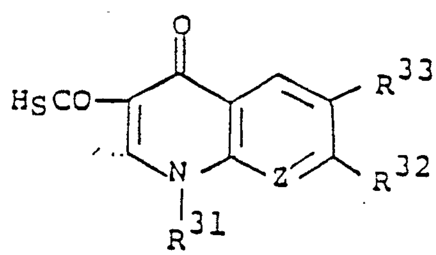

III

in der $R^{31}$, $R^{32}$ und $R^{33}$ die in Anspruch 1 gegebene Bedeutung haben,
umsetzt, oder dass man

(b) zur Herstellung einer Carbonsäure der Formel I, einen Ester der allgemeinen Formel

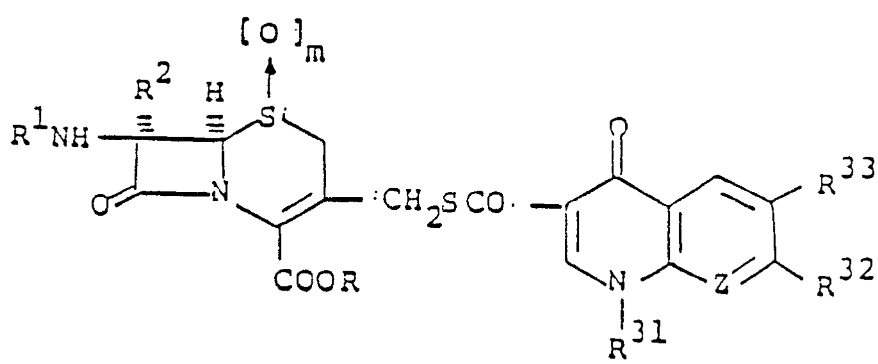

IV

in der m, $R^1$, $R^2$, $R^{31}$, $R^{32}$ und $R^{33}$ die in Anspruch 1 gegebene Bedeutung haben, und R eine Esterschutzgruppe darstellet,
in die Carbonsäure der Formel I umwandelt, oder dass man

(c) zur Herstellung einer Verbindung der Formel I, worin m 0 darstellt, eine Verbindung der allgemeinen Formel

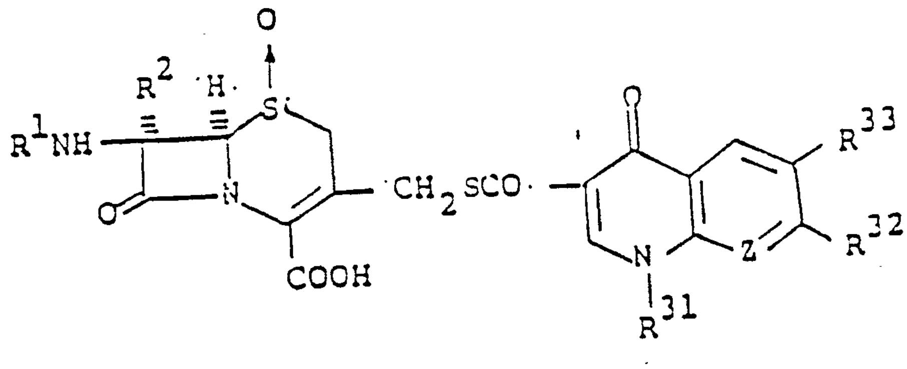

V

in der $R^1$, $R^2$, $R^{31}$, $R^{32}$ und $R^{33}$ die in Anspruch 1 gegebene Bedeutung haben,
reduziert, oder dass man

(d) zur Herstellung einer Verbindung der Formel I, worin m 1 oder 2 darstellt, oder eines ihrer Ester oder Salze, eine Verbindung der allgemeinen Formel

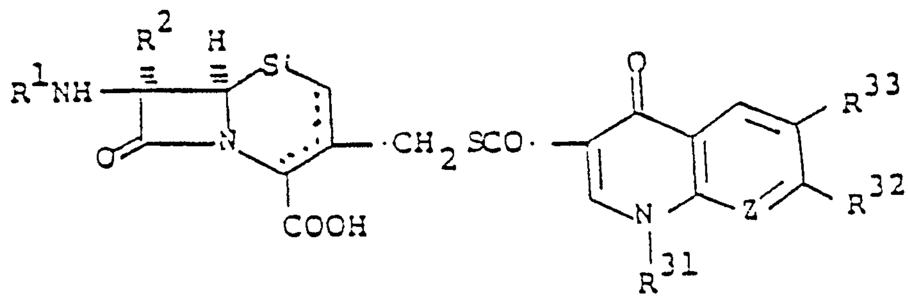

VI

in der $R^1$, $R^2$, $R^{31}$, $R^{32}$ und $R^{33}$ die in Anspruch 1 gegebene Bedeutung haben, und die gestrichelten Linien die Anwesenheit einer Δ2- oder Δ3-Doppelbindung anzeigen,
oder eines ihrer Ester oder Salze oxidiert, oder dass man

(e) zur Herstellung einer Verbindung der Formel I, worin $R^1$ einen Aminosubstituenten enthält oder eines ihrer Ester oder Salze, die Aminoschutzgruppe im Substituenten $R^{10}$ einer Verbindung der allgemeinen Formel

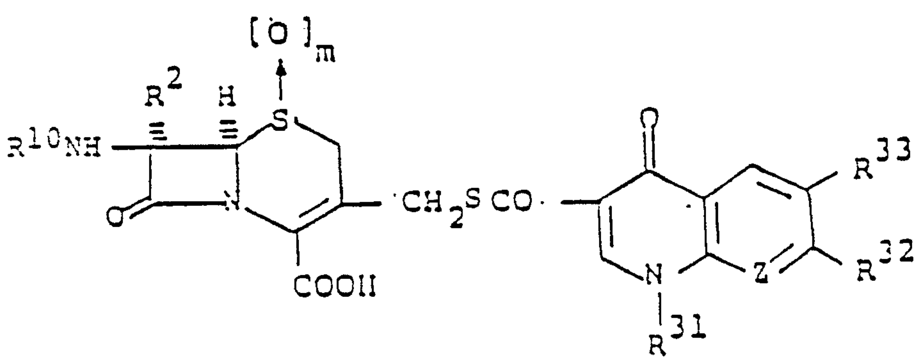

VII

in der m, $R^2$, $R^{31}$, $R^{32}$ und $R^{33}$ die in Anspruch 1 gegebene Bedeutung haben und $R^{10}$ einen eine geschützte Aminogruppe enthaltenden Acylrest darstellt,
oder eines ihrer Ester oder Salze abspaltet, oder dass man

(f) zur Herstellung eines leicht hydrolysierbaren Esters einer Verbindung der Formel I, eine Carbonsäure der Formel I einer entsprechenden Veresterung unterwirft, oder dass man

(g) zur Herstellung von Salzen oder Hydraten einer Verbindung der Formel I bzw. von Hydraten besagter Salze, eine Verbindung der Formel I in ein Salz oder Hydrat bzw. in ein Hydrat des besagten Salzes überführt.

28. Verfahren nach Anspruch 27, dadurch gekennzeichnet, dass man eine der Alternativen (a), (b), (c) und (d) ausführt.

29. Verwendung von Verbindungen gemäss einem der Ansprüche 1-22 bei der Behandlung bzw. Prophylaxe von Krankheiten.

30. Verwendung von Verbindungen gemäss einem der Ansprüche 1-22 bei der Behandlung bzw. Prophylaxe von Infektionskrankheiten.

31. Verwendung von Verbindungen gemäss einem der Ansprüche 1-22 bei der Herstellung von Arzneimitteln für die Behandlung bzw. Prophylaxe von Infektionskrankheiten.

Patentansprüche für folgende Vertragsstaaten : GR, ES

1. Verfahren zur Herstellung von Acylderivaten der allgemeinen Formel

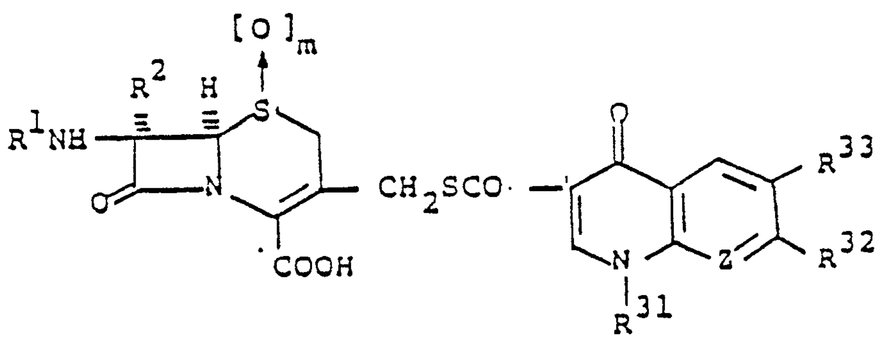

I

in der m 0, 1 oder 2, $R^1$ Wasserstoff oder eine Acylgruppe, $R^2$ Wasserstoff, niederes Alkoxy, niederes Alkylthio oder niederes Alkanoylamino, $R^{31}$ Wasserstoff, niederes Alkyl, niederes Alkenyl, $C_3$-$C_7$-Cycloalkyl, Halogen-niederes Alkyl oder Mono-, Di- oder Tri-halogenphenyl; Z $R^{30}$ -C oder Stickstoff, $R^{30}$ Wasserstoff oder Halogen, oder $R^{30}$ und $R^{31}$ zusammen eine $C_3$-$C_5$-Alkylengruppe, eine $C_2$-$C_4$-Alkylenmonooxygruppe oder eine $C_1$-$C_2$ Alkylendioxygruppe, $R^{32}$ Wasserstoff, Halogen, niederes Alkyl oder einen gegebenenfalls substituierten 5- oder 6-gliedrigen heterocyclischen Ring mit einem, zwei oder drei Sauerstoff-, Stickstoff- und/oder Schwefelatomen und $R^{33}$ Wasserstoff oder Halogen oder $R^{32}$ und $R^{33}$ zusammen eine $C_1$-$C_4$-Alkylendioxygruppe darstellen,
sowie von leicht hydrolysierbaren Estern und Salzen dieser Verbindungen und Hydraten der Verbindungen der Formel I bzw. von deren Estern und Salzen, dadurch gekennzeichnet, dass man

(a) zur Herstellung eines leicht hydrolysierbaren Esters einer Carbonsäure der Formel I, eine Verbindung der allgemeinen Formel

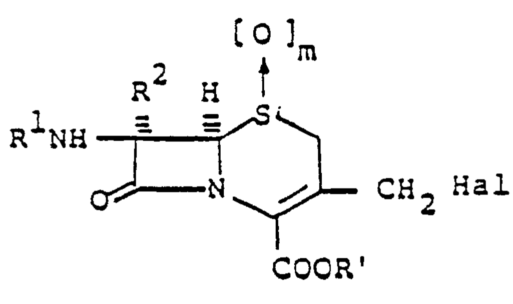

II

in der m, $R^1$ und $R^2$ die oben gegebene Bedeutung haben, Hal Halogen und $R'$ den Rest eines leicht hydrolysierbaren Esters darstellen,

mit einem Salz einer Thiocarbonsäure der allgemeinen Formel

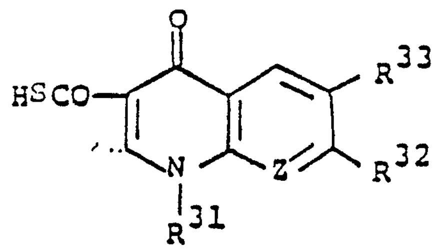

III

in der $R^{31}$, $R^{32}$ und $R^{33}$ die oben gegebene Bedeutung haben, umsetzt, oder dass man

(b) zur Herstellung einer Carbonsäure der Formel I, einen Ester der allgemeinen Formel

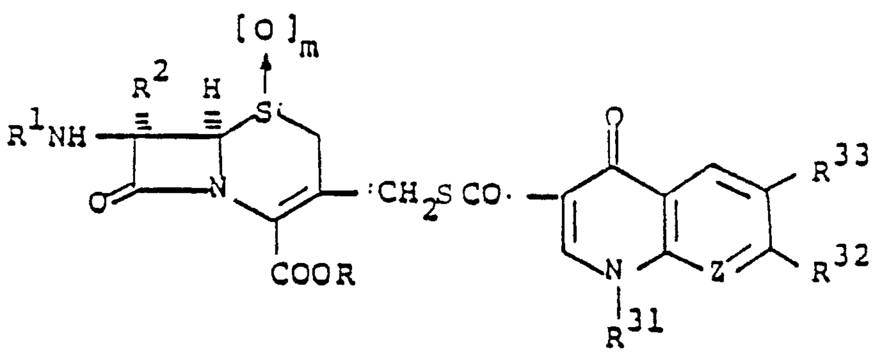

IV

in der m, $R^1$, $R^2$, $R^{31}$, $R^{32}$ und $R^{33}$ die oben gegebene Bedeutung haben, und R eine Esterschutzgruppe darstellt, in die Carbonsäure der Formel I umwandelt, oder dass man

(c) zur Herstellung einer Verbindung der Formel I, worin m 0 darstellt, eine Verbindung der allgemeinen Formel

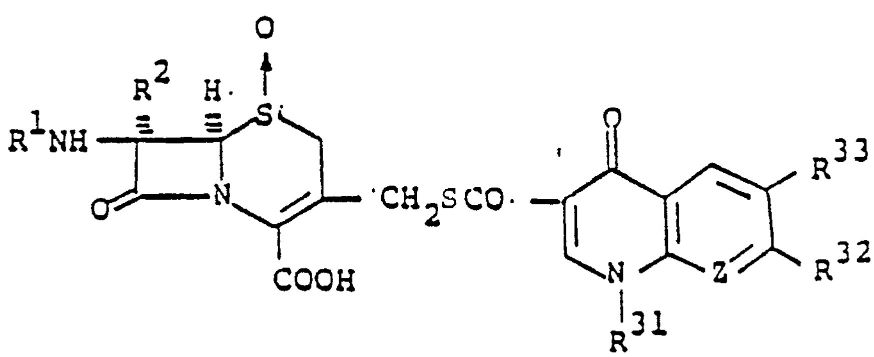

V

in der $R^1$, $R^2$, $R^{31}$, $R^{32}$ und $R^{33}$ die oben gegebene Bedeutung haben, reduziert, oder dass man

(d) zur Herstellung einer Verbindung der Formel I, worin m 1 oder 2 darstellt, oder eines ihrer Ester oder Salze, eine Verbindung der allgemeinen Formel

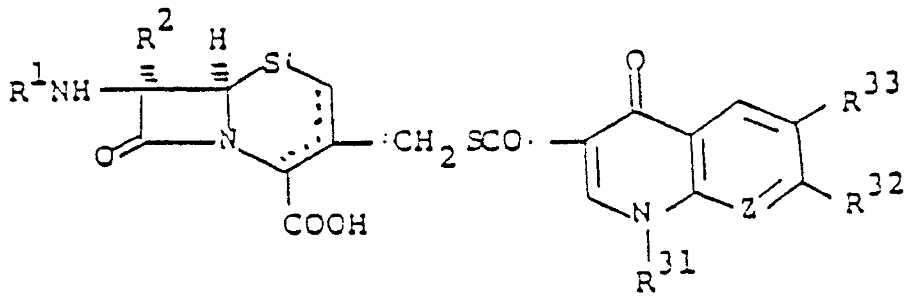

VI

in der R', $R^2$, $R^{3'}$, $R^{32}$ und $R^{33}$ die oben gegebene Bedeutung haben, und die gestrichelten Linien die Anwesenheit einer Δ2- oder Δ3-Doppelbindung anzeigen, oder eines ihrer Ester oder Salze oxidiert, oder dass man

(e) zur Herstellung einer Verbindung der Formel I, worin R' einen Aminosubstituenten enthält, oder eines ihrer Ester oder Salze, die Aminoschutzgruppe im Substituenten $R^{10}$ einer Verbindung der allgemeinen Formel

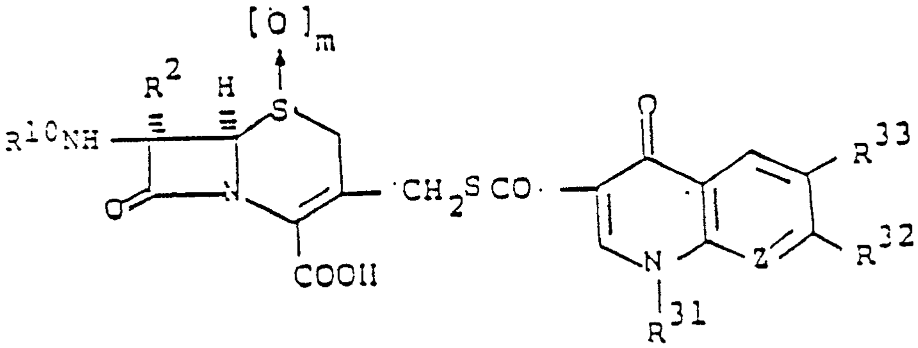

VII

in der m, $R^2$, $R^{3'}$, $R^{32}$ und $R^{33}$ die oben und $R^{10}$ einen eine geschützte Aminogruppe enthaltendes Acylrest enthält, oder eines ihrer Ester oder Salze abspaltet, oder dass man

(f) zur Herstellung eines leicht hydrolysierbaren Esters einer Verbindung der Formel I, eine Carbonsäure der Formel I einer entsprechenden Veresterung unterwirft, oder dass man

(g) zur Herstellung von Salzen oder Hydraten einer Verbindung der Formel I bzw. von Hydraten besagter Salze, eine Verbindung der Formel I in ein Salz oder Hydrat bzw. in ein Hydrat des besagten Salzes überführt.

2. Verfahren zur Herstellung von Verbindungen nach Anspruch 1, worin m 0 und $R^2$ Wasserstoff darstellt, dadurch gekennzeichnet, dass man entsprechend substituierte Ausgangsverbindungen einsetzt.

3. Verfahren zur Herstellung von Verbindungen nach Anspruch 1 oder 2, worin Z die Gruppe Z $R^{30}$-C, $R^{30}$ Wasserstoff oder Halogen, $R^{31}$ niederes Alkyl, Halogen-niederes Alkyl oder $C_3$-$C_7$-Cycloalkyl, $R^{32}$ niederes Alkyl, Piperazinyl oder niederes Alkylpiperazinyl und $R^{33}$ Wasserstoff oder Halogen darstellen, dadurch gekennzeichnet, das man entsprechend substituierte Ausgangsverbindungen einsetzt.

4. Verfahren zur Herstellung von Verbindungen nach Anspruch 3, worin $R^{30}$ Wasserstoff oder Fluor, $R^{3'}$ Aethyl, Fluoräthyl oder Cyclopropyl, $R^{32}$ Piperazinyl oder 4-Methylpiperazinyl und $R^{33}$ Wasserstoff oder Fluor darstellen, dadurch gekennzeichnet, dass man entsprechend substituierte Ausgangsverbindungen einsetzt.

5. Verfahren zur Herstellung von Verbindungen nach einem der Ansprüche 1-4, worin R' eine der folgenden Acylgruppen darstellt:

(a) eine aliphatische Gruppe der Formel

$R^5$-CO-

in der $R^5$ niederes Alkyl, $C_3$-$C_7$-Cycloalkyl, niederes Alkoxy, niederes Alkenyl, $C_3$-$C_7$-Cycloalkenyl oder Cyclohexadienyl oder durch einen oder mehrere der Reste Halogen, Cyan, Nitro, Amino, Mercapto, niederes Alkylthio oder Cyanmethylthio substituiertes niederes Alkyl oder niederes Alkenyl darstellt;

(b) eine carbocyclische aromatische Gruppe der allgemeinen Formel

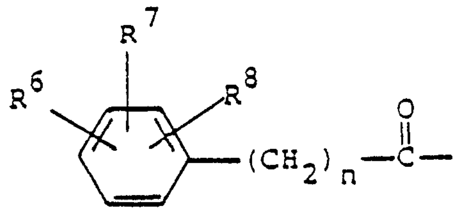

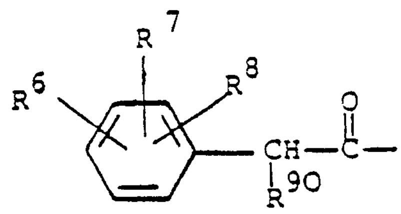

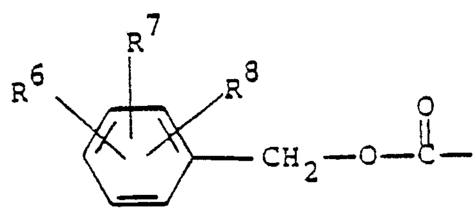

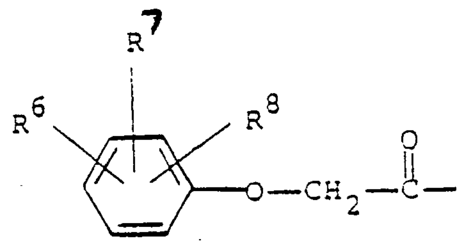

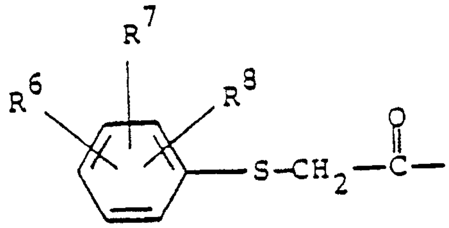

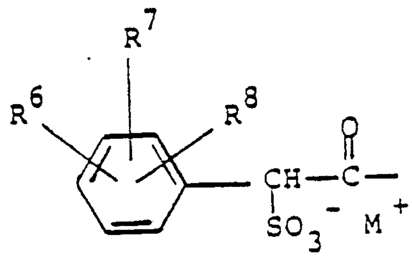

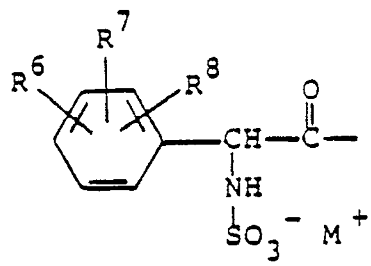

in der n 0, 1, 2 oder 3; $R^6$, $R^7$ und $R^8$ Wasserstoff, Halogen, Hydroxy, Nitro, Amino, Cyano, Trifluormethyl, $C_1$-$C_4$-Alkyl, $C_1$-$C_4$-Alkoxy oder Aminomethyl, $R^{90}$ Amino, Acylamino, Hydroxy, ein Carboxysalz, geschütztes Carboxy, Formyloxy oder Azido und M ein Kation darstellen;

(c) eine heteroaromatische Gruppe der allgemeinen Formeln

$$R^{101}-(CH_2)_n-CO-$$

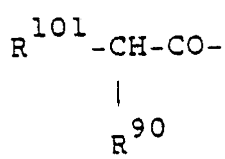

$R^{101}-O-CH_2-CO-$

$R^{101}-S-CH_2-CO-$ or

$R^{101}-CO-CO-$

in denen $R^{90}$ die oben gegebene Bedeutung hat, n 0, 1, 2 oder 3 und $R^{101}$ einen gegebenenfalls substituierten 5-, 6- oder 7-gliedrigen heterocyclischen Ring mit 1, 2, 3 oder 4 Stickstoff-, Sauerstoff- und/oder Schwefelatomen darstellt;

(d) eine [[4-Substituierte-2,3-dioxo -1-piperazinyl)carbonyl]amino]arylacetylgruppe der Formel

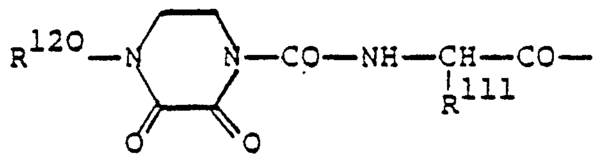

in der $R^{111}$ niederes Alkyl, Hydroxy-niederes Alkyl oder eine aromatische Gruppe der allgemeinen Formel

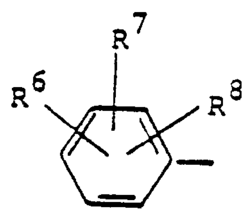

(in der $R^6$, $R^7$ und $R^8$ die oben gegebenen Bedeutung haben) oder einen wie für $R^{101}$ definierten heterocyclischen ring und $R^{120}$ gegebenenfalls durch einen oder mehrere der Reste Halogen, Cyan, Nitro, Amino und/oder Mercapto substituiertes niederes Alkyl darstellen;

(e) eine (substituierte Oxiimino)arylacetylgruppe der allgemeinen Formel

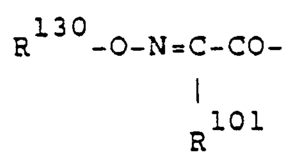

in der $R^{101}$ die oben gegebene Bedeutung hat und $R^{130}$ Wasserstoff, niederes Alkyl, $C_3$-$C_7$-Cycloalkyl, Carboxy-$C_3$-$C_7$-Cycloalkyl oder substituiertes niederes Alkyl darstellt, wobei die niedere Alkylgruppe substituiert ist durch einen oder mehrere der Reste Halogen, Cyan, Nitro, Amino, Mercapto, niederes Alkylthio, eine durch $R^{111}$ oben definierte aromatische Gruppe, Carboxy (einschliesslich dessen Salze), niederes Alkanoylamino, niederes Alkoxycarbonyl, Phenylmethoxycarbonyl, Diphenylmethoxycarbonyl, Hydroxy-(niederes Alkoxy)phosphinyl, Dihydroxyphosphinyl, Hydroxy(phenylmethoxy)phosphinyl und/oder di-(niederes Alkoxy)phosphinyl;

(f) eine (Acylamino)arylacetylgruppe der allgemeinen Formel

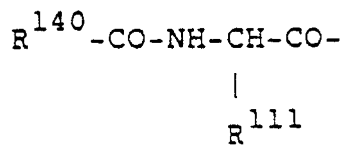

in der $R^{111}$ die oben gegebene Bedeutung hat und $R^{140}$ eine Gruppe der allgemeinen Formel

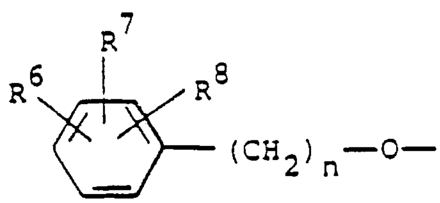

(worin $R^6$, $R^7$, $R^8$ und n die obige Bedeutung haben), Wasserstoff, niederes Alkyl, substituiertes niederes Alkyl, Amino, niederes Alkylamino, Cyanalkylamino oder Acylamino darstellt;

(g) eine [[[3-Substituierte-2-oxo -1-imidazolidinyl]carbonyl]amino]arylacetylgruppe der allgemeinen Formel

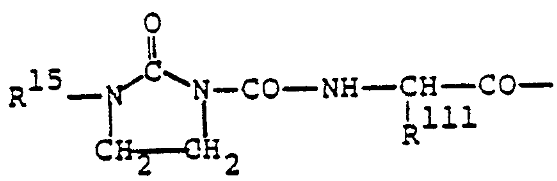

in der $R^{111}$ die oben gegebene Bedeutung hat, und $R^{15}$ Wasserstoff, niederes Alkylsulfonyl, -N = CH-$R^{111}$ (worin $R^{111}$ die oben gegebene Bedeutung hat), $R^{16}$ -CO(worin $R^{16}$ Wasserstoff oder gegebenenfalls durch Halogen substituiertes niederes Alkyl darstellt), eine wie unter $R^{111}$ oben definierte aromatische Gruppe oder gegebenenfalls durch einen oder mehrere der Rest Halogen, Cyan, Nitro, Amino und.oder Mercapto substituiertes niederes Alkyl darstellt,

dadurch gekennzeichnet, dass man entsprechend substituierte Ausgangsverbindungen einsetzt.

6. Verfahren zur Herstellung von Verbindungen nach einem der Ansprüche 1-5, worin $R^1$ eine carbocyclische aromatische Gruppe der allgemeinen Formel

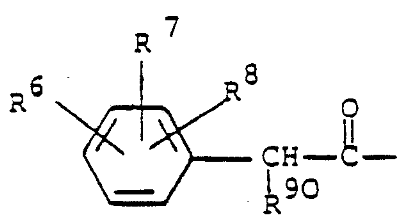

bedeutet, in der $R^{90}$ Amino, Acylamino, Hydroxy, ein Carboxysalz, Benzyloxycarbonyl, Formyloxy oder Azido und $R^6$, $R^7$ und $R^8$ Wasserstoff, Halogen, Hydroxy, Nitro, Amino, Cyan, Trifluormethyl, $C_1$-$C_4$-Alkyl, $C_1$-$C_4$-Alkoxy oder Aminomethyl darstellen,

dadurch gekennzeichnet, dass man entsprechend substituierte Ausgangsverbindungen einsetzt.

7. Verfahren zur Herstellung von Verbindungen nach Anspruch 6, worin $R^6$, $R^7$ und $R^8$ Wasserstoff und $R^{90}$ Wasserstoff oder Hydroxy darstellen, dadurch gekennzeichnet, dass man entsprechend substituierte Ausgangsverbindungen einsetzt.

8. Verfahren zur Herstellung von Verbindungen nach einem der Ansprüche 1-5, worin $R^1$ eine Acylgruppe der allgemeinen Formel

$$R^{130}-O-N{=}C-CO-$$
$$\quad\quad\quad\quad\quad\;|$$
$$\quad\quad\quad\quad\quad R^{101}$$

darstellt, in der $R^{131}$ eine gegebenenfalls durch Halogen, Hydroxy, Nitro, Amino, Cyan, Trifluormethyl, $C_1$-$C_4$-Alkyl oder $C_1$-$C_4$-Alkoxy substituierten 5-, 6- oder 7-gliedrigen heterocyclischen Ring mit 1, 2, 3 oder 4 Stickstoff-, Sauerstoff- und/oder Schwefelatomen und $R^{130}$ Wasserstoff, niederes Alkyl, $C_3$-$C_7$-Cycloalkyl, Carboxy-$C_3$-$C_7$-Cycloalkyl oder durch einen oder mehrere der Reste Halogen, Cyan, Nitro, Amino, Mercapto, niederes Alkylthio, eine durch $R^{111}$ gemäss Anspruch 5 definierte aromatische Gruppe, Carboxy (einschliesslich dessen Salze), niederes Alkanoylamino, niederes Alkoxycarbonyl, Phenylmethoxycarbonyl, Diphenylmethoxycarbonyl, Hydroxy(niederes Alkoxy)phosphinyl, Dihydroxyphosphinyl, Hydroxy-(phenylmethoxy)phosphinyl und/oder Di(niederes Alkoxy)phosphinyl substituiertes niederes Alkyl bedeuten, dadurch gekennzeichnet, dass man entsprechend substituierte Ausgangsverbindungen einsetzt.

9. Verfahren zur Herstellung von Verbindungen nach einem der Ansprüche 1-5 und 8, worin $R^1$ eine Acylgruppe der allgemeinen Formel

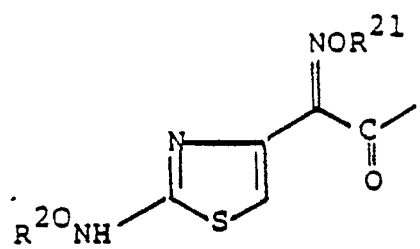

darstellt, in der $R^{20}$ Wasserstoff oder eine Aminoschutzgruppe und $R^{21}$ Wasserstoff, niederes Alkyl oder eine Gruppe der allgemeinen Formel

$$\begin{array}{c} R^{22} \\ | \\ -C-COOH \\ | \\ R^{23} \end{array}$$

bedeuten, worin $R^{22}$ und $R^{23}$ Wasserstoff, niederes Alkyl oder zusammen mit dem verbindenden Kohlenstoffatom einen 3-7-gliedrigen carbocyclischen Ring darstellen,
dadurch gekennzeichnet, dass man entsprechend substituierte Ausgangsverbindungen einsetzt.

10. Verfahren zur Herstellung von Verbindungen nach Anspruch 9, worin $R^{20}$ Wasserstoff und $R^{21}$ Methyl oder

$$\begin{array}{c} R^{22} \\ | \\ -C-COOH \\ | \\ R^{23} \end{array}$$

darstellen, worin $R^{22}$ und $R^{23}$ Wasserstoff oder Methyl bedeuten,
dadurch gekennzeichnet, das man entsprechend substituierte Ausgangsverbindungen einsetzt.

11. Verfahren zur Herstellung von Verbindungen nach Anspruch 1 der allgemeinen Formel

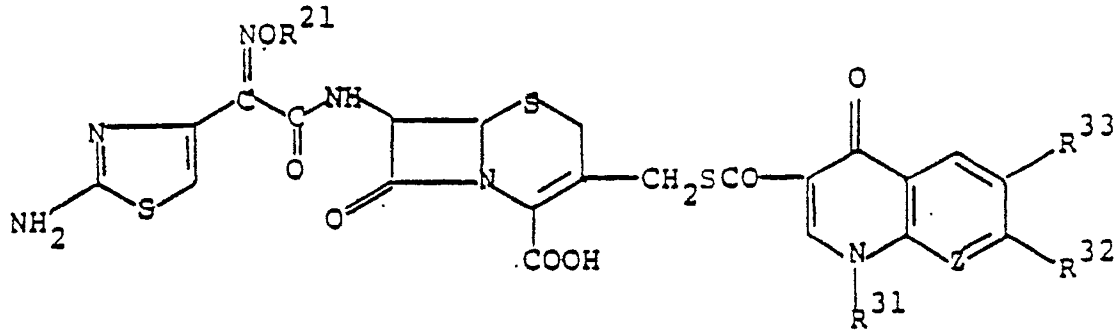

in der $R^{2'}$ die in Anspruch 9 und Z, $R^{31}$, $R^{32}$ und $R^{33}$ die in Anspruch 1 gegebene Bedeutung haben, dadurch gekennzeichnet, dass man entsprechend substituierte Ausgangsverbindungen einsetzt.

12. Verfahren zur Herstellung von Verbindungen nach einem der Ansprüche 1-11, worin $R^{2'}$ Methyl oder eine Gruppe der allgemeinen Formel

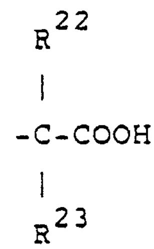

$R^{22}$ und $R^{23}$ Wasserstoff oder Methyl, Z die Gruppe $R^{30}$-C, $R^{30}$ Wasserstoff oder Halogen, $R^{31}$ niederes Alkyl, Halogen-niederes Alkyl oder $C_3$-$C_7$-Cycloalkyl, $R^{32}$ niederes Alkyl, Piperazinyl oder niederes Alkylpiperazinyl und $R^{33}$ Wasserstoff oder Halogen darstellen, dadurch gekennzeichnet, dass man entsprechend substituierte Ausgangsverbindungen einsetzt.

13. Verfahren zur Herstellung von Verbindungen nach Anspruch 12, worin $R^{30}$ Wasserstoff oder Fluor, $R^{31}$ Aethyl, Fluoräthyl oder Cyclopropyl, $R^{32}$ Methyl, 4-Methyl-piperazinyl oder Piperazinyl und $R^{33}$ Wasserstoff oder Fluor darstellen, dadurch gekennzeichnet, dass man entsprechend substituierte Ausgangsverbindungen einsetzt.

14. Verfahren zur Herstellung von Verbindungen nach Anspruch 1, gekennzeichnet als [6R-[6-alpha,7-beta(Z)]]-7-[[(2-amino -4-thiazolyl)($R^{2'}$-oxyimino)acetyl]amino] -3-[[[[6-$R^{33}$,8-$R^{30}$, 1-$R^{31}$-1,4-dihydro -7-(4-$R^{34}$-1-piperazinyl)-4-oxo-3-chinolinyl]carbonyl]thio]methyl] -8-oxo-5-thia-1-azabicyclo[4.2.0]oct-2-en-2-carbonsäuren und von deren Salzen und Hydraten dieser Carbonsäuren und Salze, worin $R^{33}$ und $R^{30}$ Wasserstoff oder Halogen, $R^{31}$ Wasserstoff, niederes Alkyl oder 2-Halogen-niederes Alkyl, $R^{34}$ Wasserstoff oder niederes Alkyl und $R^{2'}$ Wasserstoff, niederes Alkyl oder eine Gruppe der allgemeinen Formel

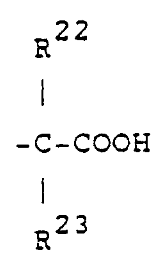

und $R^{22}$ und $R^{23}$ Wasserstoff oder niederes Alkyl bedeuten, dadurch gekennzeichnet, dass man entsprechend substituierte Ausgangsverbindungen einsetzt.

15. Verfahren zur Herstellung von Verbindungen nach Anspruch 14, worin $R^{2'}$ Methyl darstellt, dadurch gekennzeichnet, dass man entsprechend substituierte Ausgangsverbindungen einsetzt.

16. Verfahren zur Herstellung von Verbindungen nach Anspruch 15, worin $R^{33}$ und $R^{30}$ Wasserstoff oder Fluor, $R^{31}$ Aethyl oder 2-Fluoräthyl und $R^{34}$ Methyl darstellen, dadurch gekennzeichnet, dass man entsprechend substituierte Ausgangsverbindungen einsetzt.

17. Verfahren zur Herstellung von Verbindungen nach Anspruch 16, worin $R^{33}$ und $R^{30}$ Fluor und $R^{31}$ 2-Fluoräthyl darstellen, dadurch gekennzeichnet, dass man entsprechend substituierte Ausgangsverbindungen einsetzt.

18. Verfahren zur Herstellung von Verbindungen nach Anspruch 15, worin $R^{33}$ Fluor, $R^{30}$ Wasserstoff, $R^{31}$ 2-Fluoräthyl und $R^{34}$ Methyl darstellen, dadurch gekennzeichnet, dass man entsprechend substituierte Ausgangsverbindungen einsetzt.

19. Verfahren zur Herstellung von Verbindungen nach Anspruch 15, worin $R^{33}$ Fluor, $R^{30}$ Wasserstoff, $R^{31}$ Aethyl und $R^{34}$ Methyl darstellen, dadurch gekennzeichnet, dass man entsprechend substituierte Ausgangsverbindungen einsetzt.

20. Verfahren zur Herstellung von Verbindungen nach Anspruch 15, worin $R^{33}$ Fluor, $R^{31}$ 2-Fluoräthyl, $R^{30}$ Wasserstoff und $R^{34}$ Methyl darstellen, dadurch gekennzeichnet, dass man entsprechend substituierte Ausgangsverbindungen einsetzt.

21. Verfahren zur Herstellung von Verbindungen nach einem der Ansprüche 8-20, worin die Gruppen der allgemeinen Formeln

$$\begin{array}{c} N-O-R^{130} \\ \| \\ -C- \end{array} \quad \text{und} \quad \begin{array}{c} N-O-R^{21} \\ \| \\ -C- \end{array}$$

in der synisomeren Form oder als Gemische vorliegen, in denen die synisomere Form überwiegt, dadurch gekennzeichnet, dass man entsprechend substituierte Ausgangsverbindungen einsetzt.

22. Verfahren zur Herstellung von Verbindungen nach Anspruch 1, gekennzeichnet als [6R-[6α,7β(Z)]]-7-[[2-Amino-4-thiazolyl)(methoxyimino)acetyl]amino]-3-[[[[6,8-difluor-1-(2-fluoräthyl)-1,4-dihydro-7-(4-methyl-1-piperazinyl)-4-oxo-3-chinolinyl]carbonyl]thio]methyl]-8-oxo-5-thia-1-azabicyclo[4.2.0]oct-2-en-2-carbonsäure sowie von Salzen dieser Verbindung und von Hydraten dieser Verbindung bzw. Salze, dadurch gekennzeichnet, dass man entsprechend substituierte Ausgangsverbindungen einsetzt.

23. Verfahren nach einem der Ansprüche 1-22, dadurch gekennzeichnet, dass man eine der Alternativen (a), (b), (c) und (d) ausführt.

24. Verfahren zur Herstellung von pharmazeutischen Präparaten, dadurch gekennzeichnet, dass man ein in Anspruch 1 definiertes Cephalosporinderivat der Formel I oder einen leicht hydrolysierbaren Ester oder ein Salz dieser Verbindung oder ein Hydrat einer Verbindung der Formel I bzw. eines Esters oder Salzes davon als wirksamen Bestandteil mit zur therapeutischen Verabreichung geeigneten, nicht-toxischen, inerten, an sich in solchen Präparaten üblichen festen und flüssigen Trägern und oder Excipientien vermischt.

25. Verwendung von Verbindungen gemäss einem der Ansprüche 1-23 bei der Behandlung bzw. Prophylaxe von Krankheiten.

26. Verwendung von Verbindungen gemäss einem der Ansprüche 1-23 bei der Behandlung bzw. Prophylaxe von Infektionskrankheiten.

27. Verwendung von Verbindungen gemäss einem der Ansprüche 1-23 bei der Herstellung von Arzneimitteln für die Behandlung bzw. Prophylaxe von Infektionskrankheiten.